# EUROPEAN PATENT SPECIFICATION

(11) **EP 1 929 045 B1**
(45) Date of publication and mention of the grant of the patent: **21.03.2012**
(21) Application number: 06803026.1
(22) Date of filing: 06.09.2006
(51) Int. Cl.: C12Q 1/68

(54) **METHODS, COMPOSITIONS AND KITS FOR ISOTHERMAL AMPLIFICATION OF NUCLEIC ACIDS**
VERFAHREN, ZUSAMMENSETZUNGEN UND KITS ZUR ISOTHERMISCHEN AMPLIFIKATION VON NUKLEINSÄUREN
PROCEDES, COMPOSITIONS ET NECESSAIRES POUR L'AMPLIFICATION ISOTHERME D'ACIDES NUCLEIQUES

(30) Priority: 06.09.2005 US 714281 P; 29.09.2005 US 722028 P
(43) Date of publication of application: 11.06.2008
(73) Proprietor: GEN-PROBE INCORPORATED, San Diego, CA 92121-4362 (US)
(72) Inventor: BECKER, Michael, M., San Diego, California 92131 (US); LIVEZEY, Kristin, W., Encinitas, CA 92024 (US)
(74) Representative: Viering, Jentschura & Partner
(86) International application number: PCT/US2006/034663
(87) International publication number: WO 2007/030505

(56) References cited:
- EP-A- 1 686 190
- EP-A1- 0 549 107
- EP-A1- 1 020 534
- WO-A-96/01327
- WO-A2-2004/042034
- US-A- 5 624 825
- US-A1- 2004 072 164
- ROMANO J W ET AL: "NASBA A NOVEL, ISOTHERMAL DETECTION TECHNOLOGY FOR QUALITATIVE AND QUANTITATIVE HIV-1 RNA MEASUREMENTS" CLINICS IN LABORATORY MEDICINE, W.B. SAUNDERS CO., LONDON, GB, vol. 16, no. 1, 1 March 1996 (1996-03-01), pages 89-103, XP000600141 ISSN: 0272-2712
- TAYA T ET AL: "HOMOGENEOUS DETECTION OF A TARGET NUCLEIC ACID SEQUENCE BY COMBINATION OF THE INTERCALATION ACTIVATING FLUORESCENCE DNA PROBE AND THE ISOTHERMAL SEQUENCE AMPLIFICATION" NUCLEIC ACIDS SYMPOSIUM SERIES, IRL PRESS, OXFORD, GB, vol. 42, 1999, pages 51-52, XP001135170 ISSN: 0261-3166
- DETTER J C ET AL: "Isothermal strand-displacement amplification applications for high-throughput genomics" GENOMICS, ACADEMIC PRESS, SAN DIEGO, US, vol. 80, no. 6, December 2002 (2002-12), pages 691-698, XP002310697 ISSN: 0888-7543
- NOTOMI T ET AL: "Loop-mediated isothermal amplification of DNA" NUCLEIC ACIDS RESEARCH, OXFORD UNIVERSITY PRESS, SURREY, GB, vol. 28, no. 12, 15 June 2000 (2000-06-15), pages 1362-4962, XP002292090 ISSN: 0305-1048
- AMBION: "A PCR strategy for rapid generation of template DNA for synthesis of labeled RNA probes" TECHNICAL BULLETIN # 154, [Online] 26 June 2005 (2005-06-26), XP002415477 Retrieved from the Internet: URL:http://web.archive.org/web/20050726082 853/http://www.ambion.com/techlib/tb/tb_15 4.html> [retrieved on 2007-01-18]
- WHITING S H ET AL: "Strand displacement synthesis capability of Moloney murine leukemia virus reverse transcriptase." JOURNAL OF VIROLOGY AUG 1994, vol. 68, no. 8, August 1994 (1994-08), pages 4747-4758, XP002415537 ISSN: 0022-538X
- HOWARD JUDELSON: 'Guidelines for designing primers', [Online] Retrieved from the Internet: <URL:http://oomyceteworld.net/protocols/pri mer%20designing2.pdf> [retrieved on 2011-01-06]
- VINCENT R. PREZIOSO: 'General Notes on Primer Design in PCR', [Online] Retrieved from the Internet: <URL:http://mikrobi.web.elte.hu/gyakorlat/f iles/segedanyag_1.pdf> [retrieved on 2011-01-06]

## Description

### Field of the Invention

The invention relates to methods for exponential and linear amplification of nucleic acid without thermal cycling, for detecting and quantifying a nucleic add.

### Background

A common method for detecting and quantifying target nucleic acid sequences is nucleic add hybridization, in which, usually, a labeled nucleic add probe sequence complementary to the target sequence is used. The probe is mixed with a sample suspected of containing the target sequence under hybridization conditions, in which the probe hybridizes to the target sequence. Hybridization can be detected by various techniques, such as by detecting a signal from a label on a probe hybridized to the target sequence.

A target nucleic acid can be detected and quantified by amplifying the target nucleic add to detectable levels, by using any of a variety of amplification methods. Many methods use conditions that require thermal cycling, in which specific target/primer oligonucleotide hybrids are formed, complementary sequences are synthesized from the end of a primer, and double-stranded nucleic adds formed by the synthesis are denatured in a series of cycles resulting in geometric amplification of the target sequence. Examples of thermocycling amplification processes include the polymerase chain reaction (PCR), transcription amplification systems (TAS), ligase chain reaction (LCR), Random Priming Amplification (RPA), and amplification methods that use Q beta replicase, restriction endonucleases and random haxamers.

Some amplification processes for detecting and quantifying target nucleic acid sequences are conducted without temperature cycling. Some methods are conducted at temperatures that destabilize the double-stranded reaction product. Other methods uses two sets of primers, each set including at least two primers. In transcription-mediated amplification (TMA) method, two enzymes, a reverse transcriptase and a RNA polymerase, are used to produce amplification products.

### Summary of the Invention

An isothermal nucleic acid amplification method is disclosed that includes the steps of providing a reaction mixture that includes a nucleic acid template strand, extension nucleotides, a first oligonucleotide primer that contains an AT-rich sequence X and a sequence Z that is complementary to a sequence In the template strand, a second oligonucleotide primer consisting of a sequence contained in the template strand, and a nucleic acid polymerase having strand displacement activity; hybridizing sequence Z of the first oligonucleotide primer to a complementary sequence in the template strand; synthetically extending the first oligonucleotide primer from the 3' Romano et al. (Clinics in Laboratory Medicine (1996), 16(1):89-103) disclose NASBA as an isothermal detection technology for qualitative and quantitative HIV-1 RNA measurements. Using the standard NASBA protocol, reverse transcriptase generates a double-stranded DNA complement from a single-stranded RNA template in the presence of suitable primers comprising a T7 promoter primer and subsequently RNA amplicons of the double-stranded DNA are generated by the action of T7 polymerase.
Technical Bulletin #154 of Ambion discloses a PCR strategy for the generation of template DNA for synthesis of labeled RNA probes. In this connection, it is disclosed to use PCR primers that have the T7 promoter sequence appended at the 5' end in the PCR reaction so that from the thus generated DNA amplicons RNA probes can be synthesized by use of T7 RNA polymerase by *in vitro* transcription.
International patent publication WO96/01327 discloses a nucleic acid target amplification method using oligonucleotide primers consisting of a 3' portion hybridizable with the target and a 5' portion comprising an inverted repeat that can be folded into a hairpin structure.
European patent application EP 1020534 A1 discloses an isothermal process for the synthesis of nucleic acids. According to this process, if two primers for the same template strand are used to facilitate displacement, the outer primer should have a lower melting temperature than the inner primer to allow the inner primer to anneal and be extended first so that subsequently displacement can occur. The inner primer comprises a 5' target-identical sequence with a higher melting point than the target-complementary part so that the strand formed by use of this primer has self-complementarity and forms a hairpin structure. By the use of such a primer, isothermal amplification can be achieved by use of a single enzyme with strand displacement activity.
Judelson ("Guidelines for designing primers", URL http://oomyceteworld.net/protocols/primer%20designing2.pdf, version 2011-01-06) and Prezioso ("General notes on primer design in PCR", URL http://mikrobi.web.elte.hu/gyakorlat/files/segedanyag_1.pdf, version 2011-01-06) disclose general guidelines for primer design and teach away from using polyA and polyT ends in primers, as these will lead to "breathing" ends that impair amplification efficiency. terminus of sequence Z by nucleic acid polymerization to make sequence Y that is complementary to at least part of the template strand, thereby forming a first strand of a double-stranded nucleic acid that is isothermally amplified; hybridizing the second oligonucleotide primer to a complementary sequence contained in sequence Y; synthetically extending the 3' terminus of the second oligonucleotide primer by nucleic acid polymerization, thereby forming a second strand of the double-stranded nucleic acid that is isothermally amplified, in which the second strand contains an AT-rich sequence complementary to sequence X of the first oligonucleotide primer, thereby forming an AT-rich region of the double-stranded nucleic acid that is isothermally amplified; hybridizing the first oligonucleotide primer to the second strand of the double-stranded nucleic acid that is isothermally amplified when the AT-rich region of the double-stranded nucleic acid is partially opened to make the second strand accessible to the first oligonucleotide primer; and polymerizing an extension product of the first oligonucleotide primer hybridized to the second strand by using the nucleic acid polymerase having strand displacement activity, thereby displacing the first strand of the double-stranded nucleic acid and performing at least one amplification cycle under isothermal conditions on the double-stranded nucleic acid that is isothermally amplified. In a preferred embodiment, the amplification cycle also includes hybridizing the second oligonucleotide primer to the first strand that was displaced by polymerizing to form the extension product of the first oligonucleotide primer, and extending the 3' terminus of the second oligonucleotide primer by nucleic acid polymerization using the first strand as a template. In a preferred embodiment in which the nucleic acid template strand is ssRNA, the reaction mixture also includes an enzyme with reverse transcriptase (RT) activity and a means for cleaving RNA, whereby the RT activity synthetically extends the first oligonucleotide primer from the 3' terminus of sequence Z to make sequence Y in the first strand and the means for cleaving RNA degrades the ssRNA template strand. In another preferred embodiment, the nucleic acid template strand is ssDNA and the method also includes a step of chemically or physically denaturing the template strand from the first strand made by synthetically extending the first oligonucleotide primer from the 3' terminus of sequence Z by nucleic acid polymerization to make sequence Y. In another preferred embodiment in which the nucleic acid template strand is ssDNA, the method also includes providing in the reaction mixture a third oligonucleotide that includes sequence T that hybridizes to a sequence in the ssDNA template strand located 3' of the sequence to which sequence Z hybridizes; hybridizing the third oligonucleotide to the ssDNA template strand at a location 3' to the sequence to which sequence Z hybridizes; and synthetically extending the 3' end of the third oligonucleotide by nucleic acid polymerization using the polymerase having strand displacement activity, thereby displacing from the template strand the first strand synthesized by extension of the first oligonucleotide primer. In another preferred embodiment, the nucleic acid template strand is a first strand of a dsDNA and an osmolyte is provided in the reaction mixture; and the method may include an optional step of chemically or physically denaturing the dsDNA before hybridizing the first oligonucleotide primer to the first strand of the dsDNA that serves as a template for synthetically extending the - first oligonucleotide primer from the 3' terminus of sequence Z by nucleic acid polymerization to make sequence Y. In another preferred embodiment, the nucleic acid template is ssDNA having a defined 3' end and the method includes synthetically extending the 3' end of the ssDNA by nucleic acid polymerization to make an AT-rich sequence complementary to the sequence X of the first oligonucleotide primer, thus forming an AT-rich region of the double-stranded nucleic acid that is isothermally amplified. In a preferred embodiment, the nucleic acid template strand is a first strand of a dsRNA and the method includes the steps of providing an enzyme that has reverse transcriptase (RT) activity and a means for cleaving RNA, and before the hybridizing steps, chemically or physically denaturing the dsRNA to separate the first ssRNA strand that hybridizes to the first oligonucleotide primer and a second ssRNA strand that hybridizes to the second oligonucleotide primer, then hybridizing sequence Z of the first oligonucleotide primer to a complementary sequence in the first ssRNA strand and hybridizing the second oligonucleotide primer to a complementary sequence in the second ssRNA strand, using the RT activity to synthetically extend the 3' terminus of the first oligonucleotide primer hybridized to the first ssRNA strand and the 3' terminus of the second oligonucleotide primer hybridized to the second ssRNA strand, and using the means for cleaving RNA to degrade the first ssRNA strand to make sequence Y accessible to hybridization with the second oligonucleotide primer and to degrade the second ssRNA strand to make an extension product of the second oligonucleotide primer accessible to hybridization with the first oligonucleotide primer. A method is also disclosed for isothermal nucleic acid linear amplification, which includes the steps of providing a reaction mixture that includes a nucleic acid template strand, extension nucleotides, a oligonucleotide primer that contains an AT-rich sequence X and a sequence Z that is complementary to a sequence in the template strand, and a nucleic acid polymerase having strand displacement activity; hybridizing sequence Z of the oligonucleotide primer to a complementary sequence in the template strand; synthetically extending the oligonucleotide primer from the 3' terminus of sequence Z by nucleic acid polymerization to make sequence Y that is complementary to at least part of the template strand, thereby forming a first strand of a double-stranded nucleic acid that is isothermally amplified; synthesizing a second strand complementary to the first strand that includes a sequence complementary to sequence Y, a sequence complementary to sequence Z and an AT-rich sequence complementary to sequence X, thereby forming an AT-rich region of a double-stranded nucleic acid that is isothermally amplified; hybridizing the oligonucleotide primer to the second strand of the double-stranded nucleic acid that is isothermally amplified when the AT-rich region of the double-stranded nucleic acid is partially opened to make the second strand accessible to the oligonucleotide primer; polymerizing an extension product of the oligonucleotide primer hybridized to the second strand by using the nucleic acid polymerase having strand displacement activity, thereby displacing the first strand of the double-stranded nucleic acid and performing a first amplification cycle under isothermal conditions on the double-stranded nucleic acid that is isothermally amplified; and repeating the primer hybridizing, polymerizing and displacing steps in subsequent amplification cycles to result in linear amplification of a sequence in the nucleic acid template strand. Compositions for performing a nucleic acid amplification according to these methods include an oligonucleotide primer that contains an AT-rich sequence X and a sequence Z that is complementary to a sequence in the intended nucleic acid template strand and a nucleic acid polymerase having strand displacement activity, and may further include other primers, enzymes or osmolytes used in the methods. Such compositions are preferably in the form of a kit.

### Brief Description of the Drawings

FIG. 1 illustrates an isothermal process for amplifying a single-stranded RNA (ssRNA) template by using two oligonucleotides.

FIG. 2 illustrates an isothermal process for amplifying a single-stranded DNA (ssDNA) template by using two oligonucleotides.

FIG. 3 illustrates an isothermal process for amplifying a ssDNA template by using three oligonucleotides.

FIG. 4 illustrates an isothermal process for amplifying a double-stranded DNA (dsDNA) template by using two oligonucleotides.

FIG. 5 illustrates an isothermal process for amplifying a ssDNA template having a defined 3' end by using two oligonucleotides.

FIG. 6 illustrates an isothermal process for amplifying a double-stranded RNA (dsRNA) template by using two oligonucleotides.

FIG. 7A shows a breathing primer hybridized to a strand of a double-stranded nucleic acid.

FIG. 7B shows an inner primer hybridized to a strand of a double-stranded nucleic acid.

FIG. 7C shows a breathing primer hybridized to a single nucleic acid strand.

FIG. 7D shows a breathing primer and an outer primer hybridized to different strands of a double-stranded nucleic acid.

### Detailed Description

Nucleic acid amplification processes are widely used for detecting and quantifying a specific sequence in a sample. Detection and quantification of a specific sequence (i.e., a target sequence) is increasingly important for many applications, such as identifying and classifying microorganisms, diagnosing infectious diseases, detecting and characterizing genetic abnormalities, identifying genetic changes associated with cancer, studying genetic susceptibility to disease, measuring the response to various types of treatment, identifying criminal suspects, and resolving paternity disputes. Isothermal amplification methods and related processes, compositions and kits are described in greater detail below.

### Definitions

"Target nucleic acid" or "target" refers to a nucleic acid containing a target nucleic acid sequence. A target nucleic acid may be single-stranded or double-stranded, and often is DNA, RNA, a derivative of DNA or RNA, or a combination thereof. A "target nucleic acid sequence," "target sequence" or "target region" means a specific sequence comprising all or part of the sequence of a single-stranded nucleic acid. A target sequence may be within a nucleic acid template, which may be any form of single-stranded or double-stranded nucleic acid. A template may be a purified or isolated nucleic acid, or may be non-purified or non-isolated.

"Oligonucleotide" or "oligomer" refers to a polymer made up of two or more nucleoside subunits or nucleobase subunits joined together. An oligonucleotide may be DNA and/or RNA, and analogs thereof, containing sugar groups that may be ribose, deoxyribose and analogs thereof, e.g., ribonucleosides having a 2'-O-methylsubstitution to the ribofuranosyl moiety (US Pat. No. 6,130,038, Becker et al.). The nucleoside subunits may be joined by linkages such as phosphodiester linkages, modified linkages, or by non-nucleotide moieties which do not prevent hybridization of the oligonucleotide to its complementary target nucleic acid sequence. Modified linkages include those linkages in which a standard phosphodiester linkage is replaced with a different linkage, such as a phosphorothioate linkage or a methylphosphonate linkage. Nucleobase subunits may be joined, e.g., by replacing the natural deoxyribose phosphate backbone of DNA with a pseudo-peptide backbone, such as a 2-aminoethylglycine backbone which couples the nucleobase subunits by means of a carboxymethyl linker to the central secondary amine (sometimes referred to as "peptide nucleic acids" or "PNA"; US Pat. No. 5,539,082, Nielsen et al.). Other examples of oligonucleotides include nucleic acid analogs containing bicyclic and tricyclic nucleoside and nucleotide analogs (called "Locked Nucleic Acids" or "Locked Nucleoside Analogues" (LNA); US Pat. Nos. 6,083,482, Wang; 6,268,490, Imanishi et al.; and 6,670,461, Wengel et al.). Any nucleic acid analog is included in the term, provided that the modified oligonucleotide can hybridize to a target nucleic acid under stringent hybridization conditions or amplification conditions. Modified detection probe oligomers must hybridize preferentially to the target nucleic acid under stringent hybridization conditions.

Oligonucleotides of a defined sequence of nucleotides (nt) may be produced by well known techniques, e.g., by chemical or biochemical synthesis, and *in vitro* or *in vivo* expression from recombinant nucleic acids, i.e., excluding wild-type chromosomal DNA or *in vivo* transcripts thereof. Functional oligonucleotides include, e.g., detection, helper, and capture probes and amplification oligonucleotides.

"Amplification oligonucleotide," "primer" or "primer oligonucleotide" refers to an oligonucleotide capable of hybridizing to a target nucleic acid and acting as a primer and/or a promoter template (e.g., for synthesis of a complementary strand, thereby forming a functional promoter sequence) for the initiation of nucleic acid synthesis. If a primer is designed to initiate RNA synthesis, it may contain a sequence that is non-complementary to the target sequence but recognized by an RNA polymerase, e.g., bacteriophage T7, T3, or SP6 RNA polymerase. A primer may contain a 3' terminus modified to prevent or lessen the rate or amount of primer extension (US Pat. No. 5,766,849, McDonough et al.).

"AT-rich" refers to a nucleotide sequence or region that has a greater number of nucleotides or derivatives that form two or fewer hydrogen bonds in a duplex than nucleotides or derivatives that form three hydrogen bonds in a duplex, e.g., more adenine (A) and/or thymine (T) than guanine (G) and cytosine (C). AT-rich sequences contain 51 % or more bases capable of pairing with two or fewer hydrogen bonds (e.g., at least 51 % A and T), and preferably contain about 65% or more such bases, most preferably about 85% to 100% of such bases. For example, an AT-rich sequence may be a poly-A or poly-T sequence, or a sequence that contains a mixture of A and T residues that together are at least 51 % of the sequence or region referred to as AT-rich. An AT-rich region may be referred to as a "breathing region" because the region may become partially or completely single-stranded in conditions in which the remainder of the sequence remains double-stranded.

"Nucleic acid amplification" or "target amplification" means increasing the number of nucleic acid molecules having at least one target nucleic acid sequence, which may be linear or exponential amplification. Isothermal linear amplification processes amplify template nucleic acid and not amplification products under isothermal conditions, may be conducted using only one amplification primer, and generally amplify a target sequence by about 1,000 fold within one hour. Isothermal exponential amplification processes use a product of an amplification reaction as a substrate in a subsequent step in the amplification reaction that uses isothermal conditions to amplify a target sequence about 10,000-fold to 100,000-fold within one hour. "Amplification conditions" refer to the cumulative biochemical and physical conditions in which an amplification reaction is conducted, which may be designed based on well-known standard methods.

"Amplicon" refers to a nucleic acid generated In a nucleic acid amplification reaction and which is derived from a target nucleic acid. An amplicon may contain the target nucleic acid sequence or portion thereof and may be of the same or opposite sense as the target nucleic acid strand.

"Isothermal" means conducting a reaction at substantially constant temperature, i.e., without varying the reaction temperature in which a nucleic acid polymerization reaction occurs. Isothermal temperatures for isothermal amplification reactions are generally below the melting temperature (T_{m;} the temperature at which half of the potentially double-stranded molecules in a mixture are in a single-stranded, denatured state) of the predominant reaction product, i.e., generally 90ºC or below, usually between about 50ºC and 75ºC, and preferably between about 55ºC to 70 ºC, or 60 ºC to 70ºC, or more preferably at about 65ºC. Although the polymerization reaction may occur in isothermal conditions, an isothermal process may optionally include a pre-amplification heat denaturation step to generate a single-stranded target nucleic acid to be used in the isothermal amplifying step.

"Polymerase" means an enzyme capable of catalyzing template dependent oligonucleotide extension by conjugating extension nucleotides to an oligonucleotide or amplicon. In isothermal amplification processes, the polymerase generally promotes strand displacement, which refers to the ability of a polymerase to displace downstream DNA encountered during primer extension. DNA polymerases having strand displacement activity include those of phi29 DNA polymerase, DNA polymerase I, Klenow fragment, Klenow fragment (3' -> 5' exo-), DNA polymerases isolated or derived from thermophilic organisms, e.g" VENT® DNA Polymerase, 9° Nm DNA Polymerase, Therminator DNA Polymerase, *Bacillus stearothermophilus* (Bst) DNA polymerase (US Pat. Nos. 5,874,282; 6,100,078, and 6,066,483, Riggs et al.), and the large fragment of Moloney murine leukemia virus (MMLV) reverse transcriptase (RT). In preferred embodiments, a Bst DNA polymerase may be modified to reduce, inhibit, inactivate or remove its 5' exonuclease activity (i.e., 5'-exo-minus polymerase). A polymerase may have reverse transcriptase (RT) activity which catalyzes extension of a DNA complement from an RNA template (i.e., RNA directed DNA polymerase), such as in MMLV RT and avian myeloblastosis virus (AMV) RT enzymes. RT activity may be provided in a fragment of a native polymerase. Preferred polymerases include those that tolerate modified oligonucleotides and/or modified extension nucleotides when catalyzing oligonucleotide extension.

"Extension nucleotides" refer to any nucleotide capable of being incorporated into an extension product during amplification, i.e., DNA, RNA, or a derivative if DNA or RNA, which may include a label.

"Osmolyte" means a molecule that contributes to the osmotic strength of an amplification system, which is added to some preferred embodiments to preferably enhance isothermal amplification. "Means for cleaving RNA" refers to a component or condition that degrades RNA, such as by contacting RNA with base (e.g., NaOH) or one or more enzymes with RNase activity, or by providing shearing conditions (e.g., sonication). In some embodiments, means for cleaving RNA is an enzyme having RNaseH activity, which degrades RNA in an RNA/DNA duplex.

A "binding molecule" is a substance that hybridizes or otherwise binds to an RNA target adjacent to or near the 3'-end of the desired target sequence, to limit a DNA primer extension product to a desired length, i.e., to make a primer extension product having a 3'-end defined within a small range of bases. In contrast, in the absence of a binding molecule, the primer extension reaction produces an indeterminate 3' end. A binding molecule may include a nucleic acid region, e.g., DNA, RNA, DNA:RNA chimeric molecule, or analogs thereof, to serve as terminating and digestion oligonucleotides. Nucleic acid binding molecules may be modified, e.g., to include a protein or drug that binds RNA specifically to limit a DNA primer extension product to a pre-determined length.

A "terminating oligonucleotide" is an oligonucleotide that includes a sequence that is complementary to a target nucleic region near the 5'-end of the target sequence, to "terminate" extension by a polymerase of a nascent nucleic acid strand that includes a primer, thereby providing a nascent strand with a defined 3'-end. A terminating oligonucleotide hybridizes at a target position that results in the desired 3'-end, and usually includes a blocking moiety at its 3'-terminus to prevent extension of the terminating oligonucleotide. A terminating oligonucleotide may include modified structures, e.g., synthesized with at least one 2'-O-methylribonucleotide (Majlessi et al., 1988, Nucleic Acids Res. 26: 2224-9), with PNA or LNA structures (Petersen et al., 2000, J. Mol. Recognit. 13: 44-53), or joined to a protein or peptide that terminates strand extension. Terminating oligonucleotides are usually at least 10 to 50 nt or longer.

"Modifying oligonucleotide" refers to an oligomer that includes a motif that hybridizes to a sequence near the 5' or 3' end of an RNA target to terminate primer extension. When the modifying oligonucleoide hybridizes near the 5'-end of the RNA target, it facilitates termination of primer extension by means of a modifying enzyme (e.g., nuclease) to determine the 3'-terminus of the primer extension product. When the modifying oligonucleotide hybridizes near the 3'-end of the RNA target sequence, it is tethered to a specific modifying enzyme or to a chemical to terminate primer extension. One specific modifying oligonucleotide is a "digestion oligonucleotide" that refers to a DNA oligomer of six of more residues that hybridizes to the RNA template to create a RNA:DNA hybrid in which the RNA is selectively digested by RNAse of an enzyme having RNAse H activity which is tethered to the oligonucleotide.

"Detection probe" or "probe" refers to an oligonucleotide having a sequence sufficiently complementary to its target sequence to form a probe:target hybrid stable for detection under stringent hybridization conditions. A probe is typically a synthetic oligomer that may include bases complementary to sequence outside of the targeted region which do not prevent hybridization under stringent hybridization conditions to the target nucleic acid. A sequence non-complementary to the target may be a homopolymer tract (e.g., poly-A or poly-T), promoter sequence, restriction endonuclease recognition sequence, or sequence to confer desired secondary or tertiary structure (e.g., a catalytic site or hairpin structure), which may facilitate detection and/or amplification.

"Stable" or "stable for detection" means that the temperature of a reaction mixture is at least 2ºC below the melting temperature (Tₘ) of a nucleic acid duplex contained in the mixture, more preferably at least 5ºC below the Tₘ, and even more preferably at least 10ºC below the Tₘ.

"Substantially homologous," or "substantially corresponding" means an oligonucleotide has a sequence of at least 10 contiguous bases that is at least 80%, preferably at least 90% , and most preferably 100% identical to at least 10 contiguous bases in a reference sequence. Homology between sequences may be expressed as the number of base mismatches in each set of at least 10 contiguous bases being compared.

"Substantially complementary" means that an oligonucleotide has a sequence containing at least 10 contiguous bases that are at least 80%, preferably at least 90%, and most preferably 100% complementary to at least 10 contiguous bases in a target nucleic acid sequence. Complementarity between sequences may be expressed a the number of base mismatches in each set of at least 10 contiguous bases being compared. "About" refers to the nearest rounded whole number (e.g., a lower limit of 24.4 is 24), and refers to a value having an up to 10% variance of a specified value (e.g., "about" 10 nt means plus or minus 1 nt).

"RNA and DNA equivalents" means RNA and DNA molecules having the same complementary base pair hybridization properties but different sugar moieties (i.e., ribose versus deoxyribose) and known base substitutions (uracil in RNA compare to thymine in DNA).

"Hybridization" or "hybridize" refers to the ability of completely or partially complementary nucleic acid strands to come together under specified hybridization conditions in a parallel or preferably antiparallel orientation to form a stable double-stranded structure or region (sometimes called a "hybrid") in which the two constituent strands are joined by hydrogen bonds. Although hydrogen bonds typically form between adenine and thymine or uracil (A and T or U) or cytosine and guanine (C and G), other base pairs may form (e.g., Adams et al., The Biochemistry of the Nucleic Acids, 11th ed., 1992).

"Preferentially hybridize" means that under stringent hybridization conditions, oligomers can hybridize to their target nucleic acid sequence to form stable hybrids, e.g., to indicate the presence of at least one sequence or organism of interest in a sample. A probe hybridizes to its target nucleic acid specifically, i.e., to a sufficiently greater extent than to a non-target nucleic acid to accurately detect the presence (or absence) of the intended target sequence. Preferential hybridization generally refers to at least a 10-fold difference between target and non-target hybridization signals in a sample.

"Stringent hybridization conditions," or "stringent conditions" means conditions in which an oligomer hybridizes specifically to its intended target nucleic acid sequence and not to another sequence. Stringent conditions may vary depending well-known factors, e.g., GC content and sequence length, and may be predicted or determined empirically using standard methods well known to one of ordinary skill in molecular biology (e.g., Sambrook, J. et al., . 1989, Molecular Cloning, A Laboratory Manual, 2nd ed., Ch. 11, pp. 11.47-11.57, (Cold Spring Harbor Laboratory Press, Cold Spring Harbor, NY)).

"Assay conditions" mean conditions permitting stable hybridization of an oligonucleotide to a target nucleic acid, which does not require preferential hybridization of the oligonucleotide and target.

"Consists essentially of" or "consisting essentially of" referring to an oligonucleotide means that the oligonucleotide has a sequence substantially homologous to a specified sequence and may have up to four additional bases and/or two bases deleted therefrom (i.e., sequence length and variation limitations). Additions or deletions are non-material vacations of a specified sequence which do not prevent the oligonucleotide from having its claimed property, such as hybridizing specifically to its target under stringent conditions. An oligomer may have a sequence substantially similar to a specified sequence without any additions or deletions, but a probe or primer consisting essentially of a specified sequence may include other nucleic acid sequences that do not participate in or affect hybridization to the target.

"Nucleic acid duplex," "duplex," "nucleic acid hybrid" or "hybrid" refers to a stable nucleic acid structure comprising a double-stranded, hydrogen-bonded region, e.g., RNA:RNA, RNA:DNA and DNA:DNA duplex molecules and analogs thereof. Such structure may be detected by any known means, e.g., by using a labeled probe, an optically active probe-coated substrate sensitive to changes in mass at its surface (US Pat. No. 6,060,237, Nygren et al.), or binding agents (US Pat. No. 5,994,056, Higuchi).

"Antisense," "opposite sense," or "negative sense" means a nucleic acid molecule perfectly complementary to a reference, or sense, nucleic acid molecule. "Sense," "same-sense," or "positive sense" means a nucleic acid molecule perfectly homologous to a reference nucleic acid molecule.

"Derived from" means that a nucleic acid is obtained directly from an organism or is an amplification product resulting from a nucleic acid derived from an organism.

"Capture probe" refers to an oligonucleotide that binds to a target nucleic acid (preferably in a region not targeted by a detection probe) and, either directly or indirectly, attaches the target nucleic acid to a support, to isolate it from other components in a mixture, such as a sample. Preferred capture probes include a target binding region that hybridizes to the target nucleic acid and a region that binds to an immobilized probe, which may use a member of ligand-ligate binding pair (e.g., avidin-biotin) or a sequence complementary to an immobilized probe bound to a solid support. The two regions may be contiguous sequences in an oligonucleotide or joined by otherwise, e.g., via one or more optionally modified nucleotides or by a non-nucleotide linker. A capture probe may bind both the target and immobilized probe under a variety of conditions, but preferably hybridizes under stringent conditions, first to the target nucleic acid using solution phase kinetics and then to the immobilized probe (US Pat. No. 6,110,678, Weisburg et al.).

"Immobilized probe" means an oligonucleotide that joins a capture probe to a support. An immobilized probe may be joined directly or indirectly to the support by a linkage or interaction that remains stable under the conditions used to hybridize the capture probe to the target and the immobilized probe.

"Isolate" or "isolating" means that a portion of the target nucleic acid in a sample is concentrated within or on a reaction receptacle, device, or carrier (e.g., tube, cuvette, microtiter plate well, filter, membrane, slide, pipette - tip) in a fixed or releasable manner to purify the target from other components.

"Purify" or "purifying" means that one or more components of a sample are removed from other sample components. Purified components may include particles (e.g., virus) but preferably are target nucleic acids in a generally aqueous solution phase which may include other materials, e.g., proteins, carbohydrates, lipids, non-target nucleic acid and/or labeled probes. Purifying separates a target nucleic acid from about 70%, more preferably about 90% and, even more preferably, about 95% of the other sample components.

"Helper probe" or "helper oligonucleotide" refers to an oligonucleotide that hybridizes to a target nucleic acid at a locus different from that of a detection probe, to Increase the hybridization rate of the probe and target, to increase the melting temperature (Tₘ) of the probe:target hybrid, or both.

"Phylogenetically closely related" means that organisms are closely related to each other in an evolutionary sense and are expected to have a higher total nucleic acid sequence homology than organisms that are more distantly related. Organisms that occupy adjacent and next to adjacent positions on a phylogenetic tree are closely related, but organisms that occupy positions more distant than adjacent or next to adjacent positions on the tree are closely related if they have significant total sequence homology.

### Pre-Amplification and Post-Amplification Processes

Isothermal amplification processes sometimes are part of a procedure that involves pre-amplification or post-amplification steps, which may include separating a nucleic acid template from a crude or processed biological sample before amplification, detecting one or more amplification products (amplicons) of the isothermal amplification reaction, or quantifying one or more amplicons of the reaction. In embodiments that include one or more pre-amplification or post-amplification processes, reagents adapted to these processes can be provided or used together with amplification reagents (e.g., in the same containment vessel or reaction system), or be separate from the amplification reagents.

Pre-amplification methods to isolate a target nucleic acid for use in an isothermal amplification process are well known and may be combined with the isothermal amplification methods described herein. In some embodiments, one or more capture probes are used in a pre-amplification purification to separate a template nucleic acid from a sample (US Pat. Nos. 6,110,678 and 6,280,952, Weisburg et al.). The per-amplification separation process may be conducted apart from the isothermal amplification process (e.g., at a different time and/or in a different reaction vessel), or may be part of the isothermal amplification process (e.g., contemporaneous and/or within the same reaction vessel). In other embodiments, a pre-amplification purification method may rely on nonspecific binding of nucleic acids to a support (e.g., US Pat. Nos. 5,234,809, Boom et al., 6,534,262 McKernan et al., 5,705,628, Hawkins). Any well known process to purify a target nucleic acid before the isothermal amplification may be used, although it is not necessary if the target nucleic acid in a sample is sufficiently pure to allow isothermal amplification as described herein.

In some embodiments, one or more reaction products of an amplification reaction are detected and may be quantified in conjunction with the isothermal amplification process. Reaction products can include one or more synthesized strands (amplicons), one or more unreacted oligonucleotide primers, unreacted extension oligonucleotides and template. A reaction product may be detected by using a detection probe having a nucleotide sequence complementary to a sequence in the reaction product, and optionally, one or more detectable labels or groups of interacting labels. Labels are well known and any that may be detected when associated with the reaction product to be detected may be used, e.g., a radioisotope, an enzyme, enzyme cofactor, or substrate, a dye, a hapten, a luminescent compound, e.g., a chemiluminescent, fluorescent, phosphorescent or electrochemiluminescent compound, a chromophore, an auxiliary base sequence that is unable to stably hybridize to the target nucleic acid under the stated conditions, and mixtures of these. Some embodiments use an acridinium ester (AE) label, e.g., 4-(2-succinimidyloxycarbonyl ethyl)-phenyl-10-methylacridinium-9-carboxylate fluorosulfonate ("standard AE"). Groups of interacting labels useful with a probe pair (e.g., US Pat. No. 5,928,862, Morrison), or a self-hybridizing probe with interacting compounds (e.g., US Pat. No. 5,925,517, Tyagi et al.) include, e.g., enzyme/substrate, enzyme/cofactor, luminescent/quencher, luminescent/adduct, dye dimers and Förrester energy transfer pairs. An interacting luminescent/quencher pair, such as fluorescein and DABCYL, may be used. Preferred detection probe sequences are up to 100 bases long, usually 10 to 50 bases long, and preferably about18 to 35 bases long. A detection probe often contains 10 or more contiguous bases about 80% or more, 90% or more, or 100% complementarity to a region of 10 or more bases in the reaction product.

One or more helper probes may be used in a process that includes isothermal amplification, typically in a detection step. Embodiments of helper probes are oligomers up to 100 bases long, usually from 10 to 50 bases long, and often 18 to 35 bases long. Preferred embodiments contain at least 10 to 15 contiguous bases about 80% or more, 90% or more, or 100% complementary to its intended amplicon or target nucleic acid sequence, although some embodiments may not be specific for a particular sequence.

Amplification oligonucleotides are used to prime synthesis of extension products by a nucleic acid polymerase in the isothermal amplification mixtures and reactions described herein. Preferred embodiments of primers are at least 12 bases long and hybridize specifically to the intended target sequence and its ability to be extended or copied enzymatically. While primers of different lengths and base compositions may be used, preferred embodiments have target binding regions of 18 to 40 bases that specifically and stably hybridize to their intended target sequence at the temperature at which the isothermal reaction is conducted. Those skilled in art can readily design primers for an intended target sequence taking into account parameters that affect hybridization, such as Tₘ, complementarity, secondary structure, ability to form primer hybrids or otherwise result in non-specific extension (primer-dimer or non-target copying) which may affect amplification efficiency. Thus, preferred embodiments of primers have low self-complementarity or cross-complementarity, particularly at the 3' ends of the sequences.

A nucleic acid polymerase used in the isothermal amplification methods is an agent, generally an enzyme, that incorporates RNA or DNA nt, or both, into a nucleic acid polymer in a template-dependent manner, usually in a 5' to 3' direction beginning at the 3' end of a primer. Examples of nucleic acid polymerases include DNA-directed DNA polymerases, RNA-directed DNA polymerases, and RNA-directed RNA polymerases. Preferred embodiments use a polymerase enzyme isolated from a thermophilic organism, e.g., Bst DNA polymerase or a modified version of a naturally occurring thermophilic polymerase enzyme. When a nucleic acid polymerase having 5' exonuclease activity is used, an amplification oligonucleotide may include a 5' modification to prevent enzymatic digestion. Alternatively, the polymerase enzyme may be modified to inhibit or remove 5' exonuclease activity, such as by proteolysis to make an active fragment of the enzyme without nuclease activity, to eliminate the need for 5' modified primers.

Typically, during nucleic acid amplification, a nucleic acid polymerase adds nucleotides to the 3' end of a primer using the target nucleic acid strand as a template, thereby synthesizing a strand that includes a sequence partially or completely complementary to a region of the target nucleic acid. In some reactions, the two strands of a resulting double-stranded nucleic acid are separated chemically or physically to allow amplification to proceed. Alternatively, a newly synthesized strand may be made available for binding to a primer by other means, e.g., use of strand displacement or a nucleolytic enzyme to digest part or all of a strand (e.g., the template strand), to allow cycle(s) of synthesis to produce many strands containing the target sequence or its complementary sequence.

Hybridization reaction conditions (e.g., temperature, salt concentration, detergents, and other solutes in a reaction mixture), can be selected to allow oligonucleotides used in amplification and detection to preferentially hybridize to a target nucleic acid and not to non-target nucleic acids in a sample. In conditions of increased stringency (e.g., decreased salt and/or increased temperature), the extent of hybridization decreases as hydrogen bonding between paired bases in a double-stranded hybrid molecule is disrupted, i.e., referred to as "melting." Hybridization conditions affect the stability of double-stranded nucleic acids, i.e., thermal stability of an oligonucleotide:target hybrid in particular conditions is taken into account in selecting oligonucleotides specific for a target, e.g., genus-specific or species-specific probe.

Generally, stable hybrids have more contiguous, perfectly matched (i.e., hydrogen-bonded) base pairs than occur in unstable hybrids and stable hybrids will melt last when stringency increases in the hybridization conditions. A double-stranded nucleic acid region containing one or more mismatched, "non-canonical," or imperfect base pairs that result in weaker or non-existent base pairing at those positions in a hybrid may be sufficiently stable under relatively high stringency conditions to allow a hybrid to form and be detected in a hybridization assay without cross-reacting with non-target nucleic acids in a sample. Depending on the similarity of target and non-target sequences and the degree of complementarity between an oligonucleotide and the target and non-target sequences, one or more mismatches may not interfere with the ability of the oligonucleotide to hybridize specifically to its ' intended target. Oligonucleotides, particularly detection probes, are selected to maximize the difference between the Tₘ of the oligonucleotide:target hybrid and the Tₘ of a hybrid formed between the oligonucleotide and non-target sequence (e.g., rRNA or DNA encoding rRNA (rDNA) of a non-target phylogenetically most closely-related organism in a sample). Amplification oligonucleotides, capture probes and helper probes are similarly designed to preferentially hybridize to an intended target nucleic acids under specified reaction conditions. In preferred embodiments that detect a target sequence of particular organism, design strategies include alignment and comparison of related sequences to maximize homology (e.g., alignment of conserved primary sequence and conserved secondary structure elements in rRNA sequences), selection of sequences that are most unique for the intended target nucleic acid (e.g., in variable regions), and avoidance of sequences that can intramolecularly hybridize (e.g., US Pat. Nos. 5,840,488 and 5,216,143, Hogan et al., US Pat. No. 4,851,330, Kohne). An oligonucleotide's length, sequence, GC content, and thermal stability difference between probe:target hybrids versus probe:non-target hybrids are relevant factors in designing oligonucleotides. To maximize specificity of an oligonucleotide for its intended target, preferred oligonucleotides are designed to hybridize to their targets under high stringency conditions which can be predicted, estimated, or determined by using standard methods, and preferred conditions are those that maintain a hybridization duplex (*e.g.*, Sambrook et al., supra, Ch. 11). A Hybridization Protection Assay (HPA) may be used to determine Tₘ (US Pat. No., 5,283,174, Arnold et al.), the temperature at which 50% of the maximum signal remains (US Pat. No. 5,840,488, Hogan et al.).

Oligonucleotides can be synthesized by using any standard methodology (Sambrook et al., supra, Ch. 10), e.g., phosphoramidite solid-phase chemistry (Caruthers et al., 1987, Methods in Enzymol. 154:287), automated synthesis using cyanoethyl phosphoramidite (Barone et al., 1984, Nucleic Acids Res. 12(10):4051), or procedures for synthesizing oligonucleotides containing phosphorothioate linkages (e.g., US Pat. No. 5,449,769, Batt), methylphosphonate linkages (e.g., US Pat. No. 5,811,538, Riley et al.). Following synthesis, any well known method of nucleic acid purification may be used to purify the product.

An oligonucleotide, such as a detection, helper or capture probe or amplification oligonucleotide, may be modified to contain one or more chemical groups to enhance performance or facilitate characterization of amplification products. Examples include backbone-modified oligonucleotides, or those that include phosphorothioate, methylphosphonate, 2'-O-alkyl, or peptide groups to make the oligonucleotide resistant to nucleolytic activity of certain polymerases or nucleases, or may include a non-nucleotide linker between nucleotides which do not prevent hybridization and/or elongation of the oligonucleotide (e.g., US Pat. No. 6,031,091, Arnold et al.). An oligonucleotide may contain a mixture of modified and natural bases.

An amplification oligonucleotide may be 3' modified or blocked to prevent or inhibit initiation of DNA synthesis (US Pat. No. 5,554,516, Kacian et al.), e.g., by the addition of ribonucleotides, 3' deoxynucleotide residues (e.g., cordycepin), 2',3'-dideoxynucleotide residues, modified nucleotides such as phosphorothioates, and non-nucleotide linkages (US Pat. No. 6,031,091) or alkane-diol modifications (e.g., Wilk et al., 1990, Nucleic Acids Res., 18(8):2065). A modification may be a region 3' to the priming sequence that is non-complementary to the target nucleic acid sequence. A mixture of different 3' blocked promoter-primers or of 3' blocked and unblocked promoter-primers may increase the efficiency of nucleic acid amplification. An amplification primer may be 5' modified to make it resistant to 5'-exonuclease activity in some nucleic acid polymerases, e.g, by adding a non-nucleotide group to the terminal 5' nucleotide of the primer (e.g., US Pat. No. 6,031,091).

An oligonucleotide may be labeled by using any standard enzymatic or chemical method (e.g., Sambrook et al., supra, Ch. 10), during or after oligonucleotide synthesis, e.g., by using a non-nucleotide linker group. Labels include radioisotopes and non-radioactive reporting groups, including modified nucleotides, which may be introduced internally or at the end of a nucleic acid sequence. Detection methods for such labels are well known and may be readily selected by one skilled in the art dependent on the label selected. Preferred non-isotopic labels include individual or combinations of fluorophores, such as fluorescence resonance energy transfer (FRET) pairs (e.g., US Pat. No. 5,925,517, Tyagi et al.), chemiluminescent molecules, enzymes, cofactors, enzyme substrates, haptens, or other ligands. Preferred embodiments of labeled probes include a non-nucleotide linker and acridinium ester label (e.g., US Pat. Nos. 5,185,439 and 6,031,091), Arnold et al.).

Sample processing can be performed prior to amplification of a nucleic acid containing a target sequence and may be useful to discriminate a target from non-target nucleic acid present in a sample or to increase assay sensitivity. Sample processing procedures are well known and may include direct or indirect immobilization of nucleic acids from a liquid phase on a support (e.g., US Pat. Nos. 4,486,539 and 4,563,419, Ranki et al., US Pat. No. 4,751,177, Stabinsky). Any support may be used, e.g., matrices or particles, and preferred supports are magnetically charged particles to facilitate automation of the process of recovering a target nucleic acid from other sample components (e.g., US Pat. Nos. 6,110,678, 6,280,952 and 6,534,273, Weisburg et al., US Pat. No. 6,335,166, Ammann et al).

An oligonucleotide for immobilizing a target nucleic acid on a solid support may be joined directly or indirectly to the support by any linkage or interaction which is stable under assay conditions (e.g., conditions for amplification and/or detection). Such an "immobilized probe" may bind directly to the target nucleic acid or it may Include a sequence, such as a homopolymeric tract (e.g., a poly dT) or repeating sequence (e.g., AT repeat), which hybridizes to a complementary sequence in a capture probe. Direct joining, i.e., without an intermediate group, may be via a covalent linkage, chelation or ionic interaction, whereas indirect joining joins the immobilized probe to the support via linker(s), e.g., means for binding at least two different molecules into a stable complex via members of a binding partner set that specifically bind to each other. Binding partner sets are well known, e.g., receptor and ligand, enzyme and substrate, enzyme and cofactor, enzyme and coenzyme, antibody and antigen, sugar and lectin, biotin and streptavidin, ligand and chelating agent, nickel and histidine, substantially complementary oligonucleotides, and complementary nucleic acid sequences.

A preferred sample processing embodiment uses specific binding between an immobilized probe sequence and a complementary capture probe sequence, where the capture probe also contains a target binding region that hybridizes to a target nucleic acid under assay conditions. While specificity of the target binding region of the capture probe for a region of the target nucleic acid is desirable to minimize the number of non-target nucleic acids remaining from the sample after a separation step, it is not required if the capture probes are being used solely to isolate target nucleic acid. If capture probe is not employed to isolate a target nucleic acid for subsequent amplification of a target sequence, the capture probe may further include a detectable label attached within or near the target binding region, such as a substituted or unsubstituted acridinium ester. The labeled capture probe may be used in a homogeneous or semi-homogenous assay to specifically detect hybrid nucleic acids without detecting single-stranded nucleic acids, such as the capture probe.

A preferred homogenous assay embodiment uses a hybridization protection assay (HPA) in which label associated with capture probes that have not hybridized to target nucleic acids are hydrolyzed while label associated with capture probe:target hybrids are protected. This is advantageous because only a single target-specific hybridization event (capture probe:target) is necessary for target detection, rather than multiple such events (e.g., capture probe:target and probe:target or probe:amplicon), and the assay is faster and simpler to optimize because fewer oligonucleotides are used. While the target binding region of a capture probe may be less specific in alternative assay systems, It must still be rare enough to avoid significant saturation of the capture probe with non-target nucleic acids. Thus, the requirement that two separate and specific target sequences be identified in these alternative systems could place constraints on the identification of an appropriate target. By contrast, only one such target sequence is needed when the capture probe simultaneously functions as the detection probe.

A preferred assay format includes a means for detecting the presence of the target nucleic acid in the test sample, which may be accomplished by a variety of means, including those that do not require the presence of a detectable label. Preferred embodiments use a probe with a detectable label, in which the probe includes a sequence that specifically hybridizes to a target sequence in the target nucleic acid. Once a stable probe:target nucleic acid hybrid forms, which has been directly or indirectly immobilized, unbound probe is washed away or inactivated and the remaining bound probe can be detected and/or measured.

Some embodiments of sample processing systems combine detection and nucleic acid amplification. Such systems directly or indirectly immobilize a target nucleic acid by using a capture probe and the captured target nucleic acid is purified from other sample components, followed by amplification of a target sequence in the target nucleic acid to produce an amplified product that is detected; preferably in solution with a labeled probe (US Pat. No. 6,110,678, Weisburg et al.). Target nucleic acid may be immobilized during amplification or it may be eluted from the support before amplification using appropriate conditions, e.g., incubating at a temperature above the Tₘ of the capture probe:target complex and/or of the capture probe:immobilized probe complex. Preferred embodiments use immobilized and capture probes that are "capped" or blocked at their 3' termini to prevent or inhibit their use as templates by a nucleic acid polymerase, e.g., by having 3'cordycepin, 3', 2'-dideoxynucleotides, non-nucleotide linkers, alkane-diol modifications, or non-complementary residues.

### Isothermal Amplification Reactions

Isothermal amplification process embodiments are disclosed in which a double-stranded nucleic acid containing an AT-rich nucleotide sequence is contacted with oligonucleotide primers, extension nucleotides and a polymerase enzyme that has strand displacement activity under isothermal conditions. Generally, the double-stranded nucleic acid is an amplification product of a template nucleic acid that shares one or more subsequences within the nucleic acid template. Often, an AT-rich sequence in the double-stranded nucleic acid is exogenous with respect to the nucleic acid template, typically introduced by using a primer containing the AT-rich sequence. Different process paths to and from sub-processes are described below. In the disclosed isothermal amplification processes, the system generally includes contacting in a reaction mixture a double-stranded nucleic acid, extension nucleotides, one or two primers, and a polymerase enzyme that has strand displacement activity. In the processes, a first strand of the double-stranded nucleic acid to be amplified includes an AT-rich sequence X and sequence Y, in which sequence X is 5' of sequence Y and sequence Y is complementary to a sequence in the nucleic acid template. In many embodiments, the AT-rich sequence X is introduced into a first strand of the double-stranded nucleic acid to be amplified by polymerase extension of a first primer to produce sequence Y by using the target nucleic acid as the template, and a second primer hybridizes to a sequence of the first strand (in sequence Y) and is the second primer is extended polymerase activity to make the double-stranded nucleic acid to be amplified. In the subsequent isothermal amplification cycle(s), the first primer includes sequence X and a sequence Z that hybridizes to a strand of the double-stranded nucleic acid to be amplified and the second primer hybridizes to sequence Y in the complementary strand of the double-stranded nucleic acid. Amplification embodiments described herein often use an amplicon as a reactant in subsequent amplification cycle(s). In linear amplification embodiments, only a template strand is amplified, usually by using one primer.

Some embodiments of isothermal amplification disclosed herein include an osmolyte, whereas other embodiments do not include an osmolyte for amplification. An osmolyte may be added, however, to enhance amplification rates. Any osmolyte suitable for amplification may be used, and preferred embodiments use betaine or trimethylamine N-oxide. An isothermal amplification temperature is one that provides efficient amplification without varying the temperature substantially during the reaction, and preferred embodiments use about 65°C. Any polymerase having strand displacement activity may be used, and preferred embodiments use a polymerase isolated from a thermophilic organism, such as Bst polymerase.

In some embodiments, sequence X and its complementary sequence are absent from the nucleic acid template from which the double-stranded nucleic acid is amplified. In some embodiments, however, sequence X is complementary to or is substantially identical to a subsequence in the nucleic acid template. In some embodiments, a contiguous sequence of about 6 nt or more in sequence Z is not identical to a sequence in the first strand of the double-stranded nucleic acid (i.e., first strand being the strand complementary to the strand to which the primer that include sequence Z hybridizes). In such embodiments, a contiguous sequence of about 8 nt more, 10 nt or more, 12 nt or more, 15 nt or more, or 20 nt or more in sequence Z, when present, is not identical or substantially identical to a sequence in the first strand of the double-stranded nucleic acid.

Primers for amplification generally have a target hybridization region complementary or substantially complementary to a sequence of the target sequence in the template nucleic acid. A primer is usually 100 nt or fewer, usually in a range of about 15 to 80 nt. Primers often have a target hybridization region about 8 to 40 nt. Preferred primers contain a target hybridization region of contiguous 8 nt or more that are about 80%, 90%, or 100% complementary to a sequence in the target nucleic acid of contiguous 8 nt or more. Typically, sequence X in the first primer is about 8 to 40 nt long, and may be about 65% to 100% AT-rich, preferably about 85% to 100% AT-rich. Often sequence Y, to which the second primer hybridizes, is directly adjacent to sequence X but may be separated from sequence X by one or more bases. Typically, sequence Z in the first primer is about 8 to 40 nt long, The first and second primers, or other oligonucleotides used in the amplification process usually is DNA, but may be RNA or contain one or more nucleotide derivatives having a modified base, sugar or backbone. A primer may include a promoter sequence. The 5' terminus of sequence Z may be located 5' of the 3' terminus of the nucleic acid to which it hybridizes, past the 3' terminus of the nucleic acid to which it hybridizes (i.e., some of sequence Z overhangs), or may be flush with the 3' terminus of the nucleic acid to which it hybridizes.

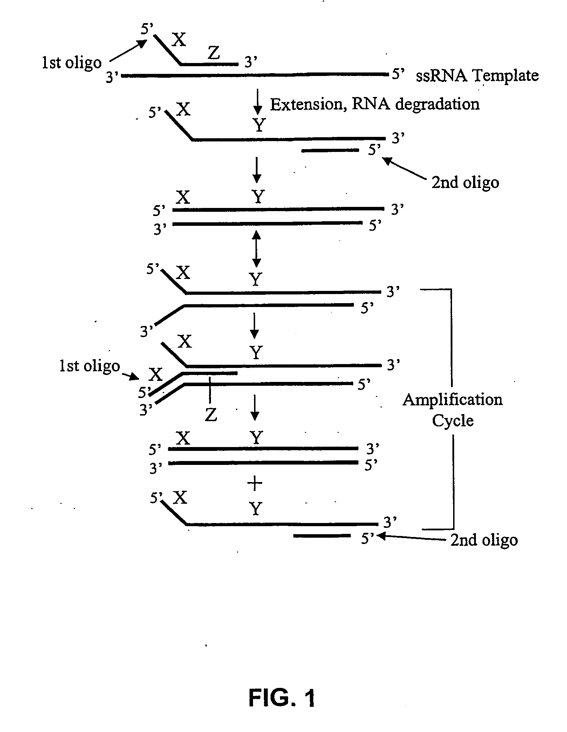

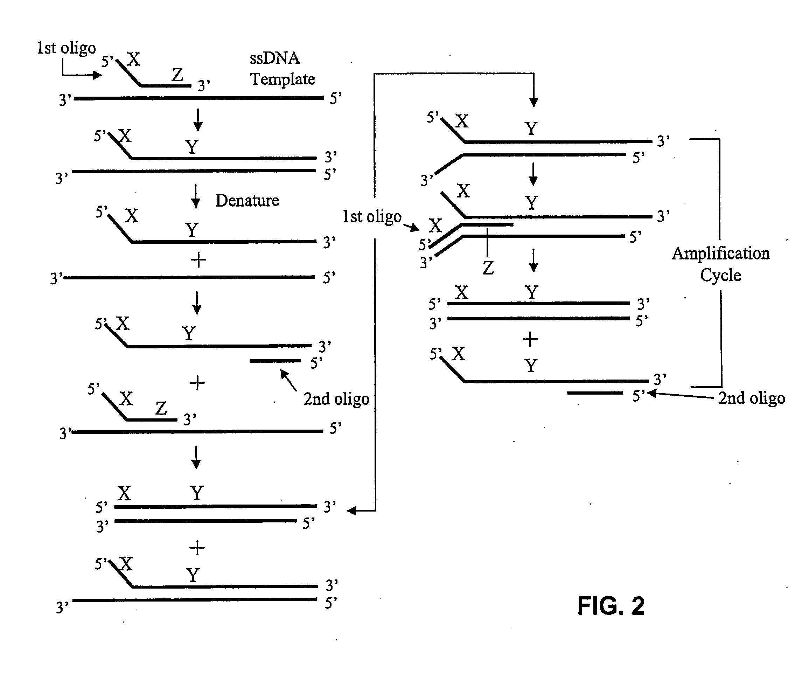

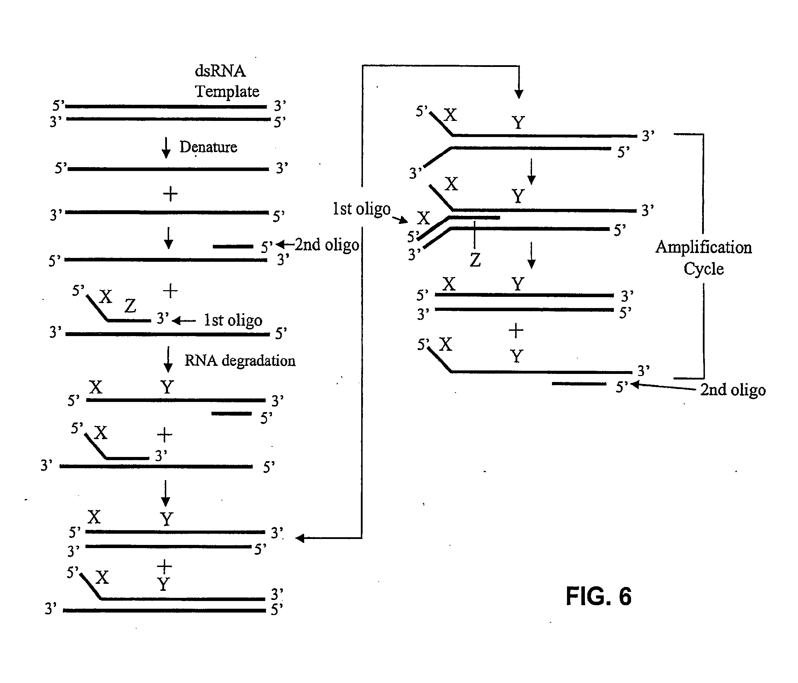

In some embodiments, the nucleic acid template from which one or both strands in the double-stranded nucleic acid are synthesized is single-stranded, such as single-stranded RNA (ssRNA, see FIG.1) or single-stranded DNA (ssDNA, see FIG. 2). In embodiments where the nucleic acid template is single-stranded, the template sometimes is a dissociation product of a double-stranded nucleic acid. A double-stranded nucleic acid may be dissociated by contacting it with chemical denaturants (e.g., urea, immidazol or formamide) and then diluting the mixture or contacting it with hydroxyl ions (e.g., from NaOH or KOH) and then neutralizing the mixture, contacting it with an osmolyte, and/or by using standard heat denaturation (see FIG. 6). If heat denaturation is used to make single-stranded template, heat is introduced before the Isothermal amplification occurs. In isothermal amplification embodiments that use RNA templates, the system may also include an enzyme having reverse transcriptase (RT) activity and a means for cleaving the single-stranded RNA template, in which the double-stranded nucleic acid that is amplified is a product of the RT extending the first primer hybridized to the ssRNA template and the polymerase having strand displacement activity extending the second primer hybridized to a first synthetic strand made in the process. In some embodiments, the RT and RNA cleaving activities are in the same enzyme.

In some embodiments, the isothermal amplification process also includes a binding molecule that binds to the nucleic acid template and limits extension of the first primer to a position before the 5' end of the nucleic acid template. Such binding molecules may bind to a ssRNA or ssDNA target template and may be a terminating or modifying oligonucleotide that hybridizes to a nucleic acid template. A binding molecule may include a nuclease activity. Embodiments include an oligonucleotide that is a peptide nucleic acid (PNA), locked nucleic acid (LNA), and those that include one or more 2'-O-methyl ribonucleotides.

In isothermal amplification embodiments in which a ssDNA is the template, it may be the product of heat denaturation of a double-stranded DNA (dsDNA). In some embodiments, the first primer hybridizes to a ssDNA template and the double-stranded nucleic acid that is amplified is a product of the polymerase extending the first and second primers. Some embodiments include a step of raising the temperature of the system sufficiently to denature a double stranded nucleic acid made up of a ssDNA template and an extension product of the first primer and then cooling to a temperature that does not denature a double-stranded nucleic acid made up of the extension products of the first and second primers (see FIG. 2). That is, the raised temperature denatures the dsDNA to generate the template and isothermal amplification steps are performed thereafter. An osmolyte may be included in an isothermal amplification reaction mixture that amplifies a ssDNA template sequence.

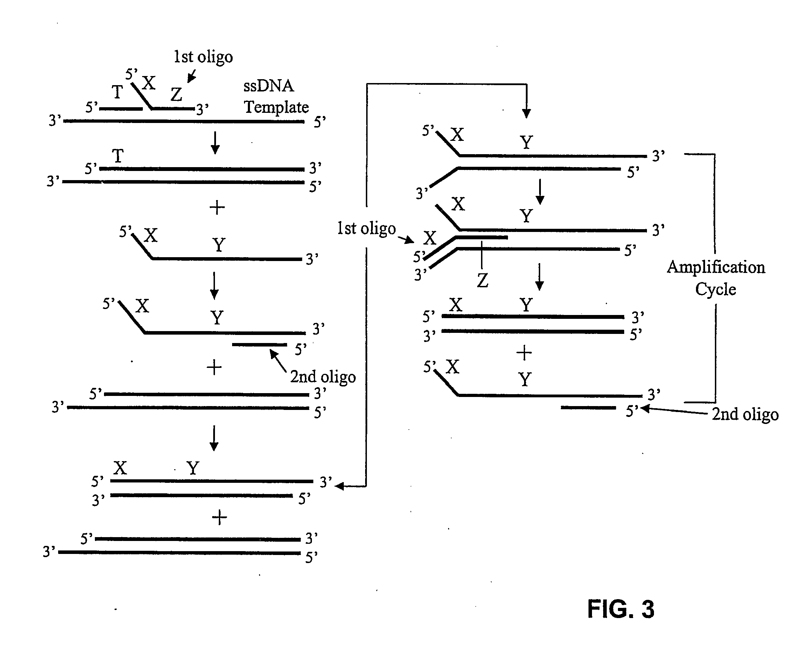

In some embodiments, the first primer having sequence X hybridizes to a ssDNA template and the 3' terminus of the ssDNA template is not extended (see FIG. 6). In some embodiments, the system includes a third oligonucleotide that includes sequence T that hybridizes to a subsequence in the ssDNA template 3' of the sequence to which sequence Z in the first primer hybridizes (see FIG. 3). The third oligonucleotide usually contains100 nt or less, and preferably contains between about 15 to 80 nt. Sequence T may be about 8 to 40 nt and may contain a target hybridization region having a contiguous sequence of about 8 nt or more that are about 80%, 90%, or 100% complementary to a contiguous target nucleic acid sequence of 8 nt or more. In some embodiments, the primer that contains sequence X also includes a sequence 5' of sequence X that hybridizes to a sequence in the ssDNA template. For example, the 5' end of the oligonucleotide that contains sequence T may be linked to the 5' end of the oligonucleotide that contains sequences X and Z via a linker of any length, preferably about 1 to 50 nt. Thus, a first primer embodiment may contain sequences T, X and Z.

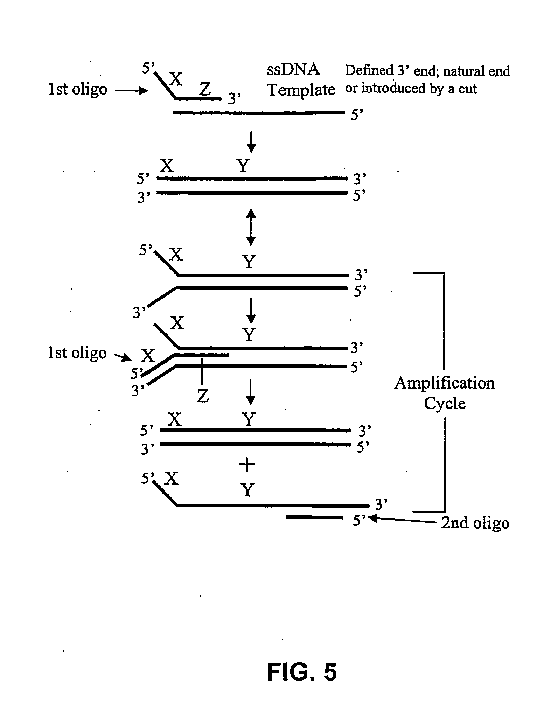

The first primer may include a sequence Z that hybridizes to the ssDNA template, whereby the 3' terminus of the ssDNA template is extended (see FIG. 5). This orientation may result from hybridization of a primer to a single-stranded template or by a restriction digest of double-stranded template.

The nucleic acid template may be a double-stranded nucleic acid in some isothermal amplification embodiments, such as in those that use a dsDNA template, dsRNA template, or a double-stranded DNA/RNA hybrid template. Such a system may include an osmolyte. In some embodiments, the first primer hybridizes to a strand of the double-stranded nucleic acid template, and the double-stranded nucleic acid that is amplified is a product of the polymerase extending the first and second primers. In some embodiments involving a dsRNA template, the method may include raising the temperature of the system sufficiently to denature the dsRNA template, and then cooling to a temperature that does not denature a double-stranded nucleic acid having one strand of the dsRNA template and an extension product of the first primer. Such embodiments include a means for cleaving the ssRNA template.

Referring to FIG.1, in one embodiment of an isothermal process for amplifying a double-stranded nucleic acid, the reaction mixture includes a ssRNA template, extension nucleotides, first and second oligonucleotide primers, a reverse transcriptase (RT) activity, means for cleaving RNA, and a polymerase having strand displacement activity. The first primer includes an AT-rich sequence X and sequence Z, in which sequence Z hybridizes to the ssRNA template and is extended to make sequence Y, while RNA degradation removes the ssRNA template strand. The extension of the first primer thus forms a first strand of the double-stranded nucleic acid that is amplified. The second primer hybridizes to sequence Y in the first strand and is extended, thus forming a second strand of the double-stranded nucleic acid that is amplified. In the amplification cycle, the AT-rich sequence X of the first strand and its complementary AT-rich sequence of the second strand may open ("breath") and/or the first primer may bind to the complementary AT-rich sequence of the second strand (via strand invasion) which results in a system that isothermally amplifies the double-stranded nucleic acid using the first and second primers in at least one subsequent polymerization cycle. Sequence X and its complementary sequence may be absent from the ssRNA template, i.e., sequence X is provided by the first primer sequence. In related linear amplification embodiments, ssRNA is amplified without the second primer in a process that uses a reaction mixture that includes the ssRNA template, extension nucleotides, a first oligonucleotide primer, RT activity and a polymerase having strand displacement activity. In this embodiment, the first primer includes an AT-rich sequence X and sequence Z, in which sequence Z hybridizes to the ssRNA template and is extended to make sequence Y in the first strand extension product. A second strand complementary to the first strand extension product may be synthesized by RT, e.g., by formation of a hairpin turn, without use of a second primer. Then, the process isothermally amplifies the ssRNA using the first primer in at least one subsequent polymerization cycle. Such linear amplification embodiments may be conducted with or without including an osmolyte and with or without including a denaturant.

Referring to FIG. 2, another embodiment of an isothermal amplification process is illustrated, which amplifies a double-stranded nucleic acid in a reaction mixture that includes a ssDNA template, extension nucleotides, first and second oligonucleotide primers ("1^{st} oligo" and "2^{nd} oligo"), and a polymerase having strand displacement activity. The reaction mixture may also Include an osmolyte. The first primer includes an AT-rich sequence X and nucleotide sequence Z which hybridizes to the ssDNA template and is extended to make sequence Y, thus forming a first strand of the double-stranded nucleic acid that is amplified. Sequence X or its complementary sequence may be absent from a subsequence in the ssDNA template. The 3' terminus of the ssDNA template is not extended in the embodiment illustrated. The template/first strand double-stranded nucleic acid is denatured partially or completely (e.g., by physical or chemical means). In an embodiment that uses physical means to partially or completely separate the strands of the double-stranded nucleic acid, the process includes raising the temperature of the system sufficient to denature a double-stranded nucleic acid made up of the ssDNA template and an extension product of the first primer and then cooling the system to a temperature that does not denature a double-stranded nucleic acid made up of the extension product of the first primer and an extension product of the second primer. The second primer hybridizes to nucleotide sequence Y in the first strand and the second primer is extended, thus forming a second strand of the double-stranded nucleic acid that is amplified. The AT-rich X sequence and its complementary AT-rich sequence in the double-stranded nucleic acid that is amplified is accessible to the first primer (as described for FIG. 1) and the isothermal amplification cycle of the double-stranded nucleic acid proceeds by using the first and second oligonucleotide primers substantially as described for FIG.1, in at least one subsequent polymerization cycle. In related linear amplification embodiments, ssDNA is amplified without the second oligonucleotide primer by contacting together a ssDNA template, extension nucleotides, a first oligonucleotide primer and a polymerase having strand displacement activity, wherein the first oligonucleotide primer includes an AT-rich sequence X and sequence Z, in which sequence Z hybridizes to the ssRNA template and is extended to make sequence Y. The process of isothermally amplifying the ssDNA uses the first oligonucleotide primer in at least one subsequent polymerization cycle. The linear amplification embodiments may be conducted with or without an osmolyte or denaturation step.

Referring to FIG. 3, an embodiment of an isothermal process for amplifying a double-stranded nucleic acid is illustrated that uses a reaction mixture that includes a ssDNA template, extension nucleotides, first, second and third oligonucleotides, and a nucleic acid polymerase having strand displacement activity. The first primer ("1^{st} oligo") includes an AT-rich sequence X and sequence Z, in which sequence Z hybridizes to the ssDNA template and is extended by the polymerase to make nucleotide sequence Y, thus forming a first strand of the double-stranded nucleic acid that is amplified. The second primer ("2^{nd} oligo") hybridizes to a portion of sequence Y in the first strand and is extended, thus forming a second strand of the double-stranded nucleic acid that is amplified. The third oligonucleotide includes a sequence T that hybridizes to a subsequence in the ssDNA template located 3' of the sequence to which sequence Z in the first primer hybridizes. When the third oligonucleotide, which may be referred to as a displacing primer, is synthetically extended by the polymerase, its extended product displaces the extension product made from the first primer from the template strand. The AT-rich region in the double-stranded nucleic acid (made up of the X sequence and its AT-rich complementary sequence) partially opens making one strand of the double-stranded nucleic acid accessible to hybridization and strand invasion by the first primer that contains sequence X, thus leading to polymerization and strand displacement resulting in at least one amplification cycle for isothermal amplification of the double-stranded nucleic acid by using the first and second primers (illustrated in the right side of FIG. 3). Sequence X or its complementary sequence may be absent from the ssDNA template. In some embodiments, the first primer and the third oligonucleotide are linked at their 5' ends so that the first primer includes sequence T.

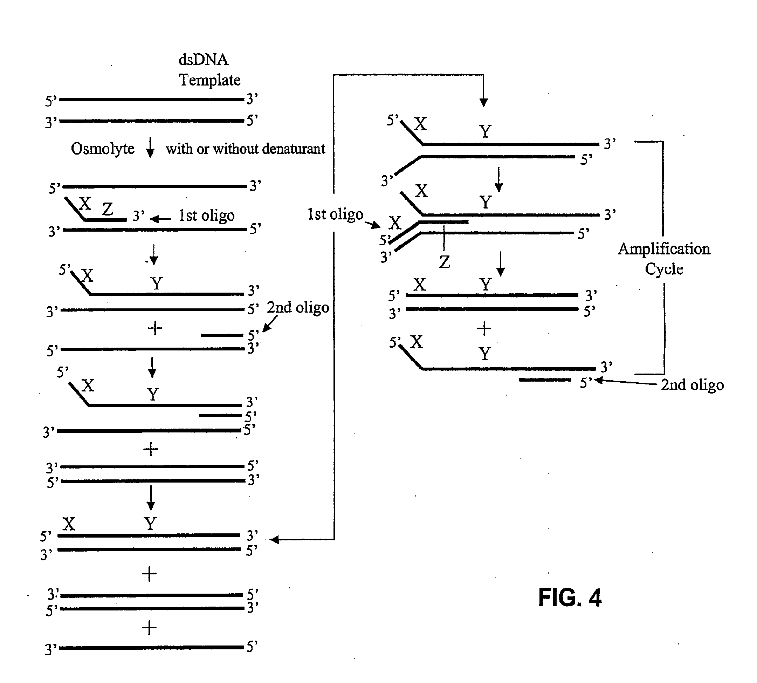

Referring to FIG. 4, another embodiment of an isothermal process for amplifying a double-stranded nucleic acid is illustrated in which the target nucleic acid is a double-stranded nucleic acid, such as a dsDNA template. In this system, the reaction mixture contacts the double-stranded nucleic acid template with extension nucleotides, first and second oligonucleotide primers ("1^{st} oligo" and "2^{nd} oligo" respectively), and a polymerase enzyme that has strand displacement activity. The reaction mixture may also include and osmolyte with or without a chemical denaturant. The first primer includes an AT-rich sequence X and sequence Z, in which sequence Z hybridizes to a strand of the double-stranded nucleic acid template and is extended by the polymerase to make sequence Y. Thus, a first strand of the double-stranded nucleic acid that is amplified is formed. The second primer hybridizes to a portion of sequence Y and is extended to form a second strand of the double-stranded nucleic acid that is amplified. The double stranded nucleic acid that is amplified includes a 5' AT-rich X sequence on one strand and a 3' complementary AT-rich sequence on the complementary strand, and those AT-rich sequences may become partially single-stranded in the amplification cycle (illustrated in the right hand portion of FIG. 4), allowing access to the first primer (that contains the X sequence) and strand displacement by the polymerase to make complementary strand, releasing the other strand of the double-stranded nucleic acid to hybridize with the second primer which is extended by the polymerase. Thus, the system isothermally amplifies the double-stranded nucleic acid by using the first and second primers in at least one subsequent polymerization cycle. Although FIG. 4 illustrates the double-stranded nucleic acid template as a dsDNA, it may alternatively be a dsRNA or a DNA/RNA hybrid. Sequence X and its complementary sequence may be absent from the double-stranded nucleic acid template because it is introduced into the system by the first primer. In a related embodiment for linear amplification, DNA is amplified without the second primer in a process which includes contacting a double-stranded nucleic acid template, extension nucleotides, a first oligonucleotide primer and a polymerase enzyme having strand displacement activity in a reaction mixture. The first primer includes an AT-rich sequence X and sequence Z and is extended to make sequence Y as described above. The second primer hybridizes to a portion of sequence Y and is extended to make the double-stranded nucleic acid that is amplified, as described above. During the amplification cycle, however, only the first primer is used to isothermally amplify one strand of the double-stranded nucleic acid in at least one subsequent polymerization cycle. Linear amplification embodiments may be conducted with or without use of an osmolyte or denaturant in the reaction mixture.

Referring to FIG. 5, another embodiment of an isothermal process for amplifying a double-stranded nucleic acid is illustrated in which the template is a ssDNA strand. The ssDNA may have a defined 3' end which is either a natural end of a DNA strand or an end introduced by a cut, such as resulting from a chemical, mechanical or enzymatic process. In this system, the reaction mixture contacts the ssDNA template, extension nucleotides, first and second oligonucleotide primers, and a polymerase enzyme having strand displacement activity, with or without an osmolyte or denaturant. The first primer ("1^{st} oligo") includes an AT-rich sequence X and sequence Z, in which sequence Z hybridizes to the ssDNA template. The first primer is extended by the polymerase to make nucleotide sequence Y, thus forming a first strand of the double-stranded nucleic acid that is amplified. The 3' end of the ssDNA template is extended by the polymerase when the first oligonucleotide primer hybridizes to the ssDNA (i.e., using the first primer as a template), thus forming a second strand of the double-stranded nucleic acid that is amplified. The double-stranded nucleic acid includes a double-stranded AT-rich region made up of the X sequence and it complementary sequence. The AT-rich region of the double-stranded nucleic acid may "breath" or become partially open allowing strand invasion by the first primer which is extended by the polymerase that has strand displacement activity, thus making a new complementary strand and allowing the second primer ("2^{nd} oligo") to hybridize to a portion of sequence Y and be extended during amplification. Thus, isothermal amplification of the double-stranded nucleic acid uses the first and second oligonucleotide primers in at least one subsequent polymerization cycle. Sequence X and its complementary sequence may be absent from the ssDNA template. In related embodiments for linear amplification, the ssDNA is amplified without the second oligonucleotide primer by contacting in a reaction mixture the ssDNA template, extension nucleotides, a first oligonucleotide primer and a polymerase enzyme having strand displacement activity, with or without an osmolyte or denaturant. The first primer includes the structural features and functions as described above to make sequence Y and the 3' end of the ssDNA template is extended when the first oligonucleotide primer hybridizes as described above. In at least one subsequent polymerization cycle, the ssDNA sequence is isothermally amplified by using only the first oligonucleotide primer, i.e., omitting the "2^{nd} oligo" illustrated in FIG. 5.

Referring to FIG. 6, another embodiment of an isothermal process for amplifying a double-stranded nucleic acid is illustrated that includes contacting in a reaction mixture a dsRNA template, extension nucleotides, first and second oligonucleotide primers, a means for cleaving RNA, and a polymerase enzyme that has strand displacement activity. The dsRNA is denatured into single strands, either by using standard physical and/or chemical methods. The first primer ("1^{st} oligo") includes an AT-rich sequence X and sequence Z. The first primer hybridizes to one strand of the dsRNA template via sequence Z and is extended to make nucleotide sequence Y, thus forming a first strand of the double-stranded nucleic acid that is amplified. The second primer ("2^{nd} oligo") hybridizes to a portion of sequence Y in the newly synthesized first strand and is extended, thus forming a second strand of the double-stranded nucleic acid that is amplified. The second primer may also hybridize to the other strand that results from denaturing the dsRNA template and be extended by the polymerase to make a double-stranded nucleic acid that does not include an AT-rich sequence unless the dsRNA template itself contained an AT-rich region. To separate the strands of the double-stranded nucleic acids, the process may raise the temperature of the system sufficiently to denature the dsRNA template and then cool the system to a temperature that does not denature a double-stranded nucleic acid made up of one strand of the dsRNA template and an extension product of the first oligonucleotide primer. For those double-stranded nucleic acids that contain an AT-rich region (e.g., defined by the X sequence of the first primer and its complementary AT-rich sequence), the AT-rich region may become accessible to strand invasion by the first primer (e.g., by partial opening of the double-stranded AT-rich region) and the polymerase having strand displacement activity then extends the 3' end of the first primer displacing the other strand of the duplex making it accessible to hybridization with the second primer. Thus, the double-stranded nucleic acid is amplified under isothermal conditions by using the first and second primers in at least one amplification cycle (illustrated in the right hand portion of FIG. 6). Sequence X and its complementary sequence may be absent from the dsRNA template, in which case, the system may include an osmolyte and/or denaturant. In related linear amplification embodiments, RNA is amplified without the second primer (i.e., omitting the "2^{nd} oligo" illustrated in FIG. 6) in a process that includes contacting in a mixture a dsRNA, extension nucleotides, the first oligonucleotide primer (as described above) and a polymerase enzyme that has strand displacement activity, with or without an osmolyte or denaturant. The first primer functions as described above to make a nucleic acid strand complementary to one strand of the dsRNA, i.e., a first synthetic strand that includes AT-rich sequence X, sequence Z and sequence Y. The polymerase makes a second synthetic sequence complementary to the first synthetic strand (e.g., by polymerization following the first strand forming a hairpin turn which allows the first synthetic strand to act as a template). The resulting double-stranded nucleic acid contains an AT-rich region (defined by sequence X and its complementary AT-rich sequence) which becomes accessible to strand invasion by the first primer as described above. The polymerase then extends the 3' end of the first primer in at least one subsequent polymerization cycle to isothermally amplify the target in a linear manner.

Kits containing reagents for performing isothermal amplification of a target nucleic acid sequence using the methods described herein may include one or more amplification oligonucleotides as described herein, and may include additional materials for isothermal amplification, such as one or more of the following components: capture probes, supports, helper probes, detection probes, binding molecules, terminating, modifying or digestion oligonucleotides, and osmolytes. Kits may include an apparatus for detecting a detection probe.

Many embodiments of the isothermal amplification methods include one or more osmolytes in the reaction mixture. Preferred osmolytes include betaine and/or trimethylamine N-oxide (TMAO). One or more osmolytes may be included, preferably at a concentration that mimics physiological concentrations, e.g., about 0.25M TMAO or about 1 M betaine. Although not wishing to be bound to a particular theory or mechanism, an osmolyte in a reaction may interact with a polymerase to facilitate strand "breathing" which may not result in strand dissociation. Osmolytes that enhance isothermal amplification may be identified by routine testing that compares results of amplification assays that test different osmolytes compared to a control reaction that does not include the osmolye, and selecting an osmolyte that enhances amplification.

Some embodiments do not include an osmolyte in the isothermal amplification reaction and, instead, a first oligonucleotide primer invades a double-stranded nucleic acid having a complementary breathing end, and the strand displaced upon extension of the first primer hybridizes to a second oligonucleotide primer (e.g., see FIG. 1). Related embodiments include an osmolyte in the reaction, in which the first and second oligonucleotide primers simultaneously invade the double-stranded nucleic acid and become extended during the isothermal amplification step.

A detection probe may be used to detect amplification products by hybridizing to the amplification product and producing a detectable signal. A detection probe may be used simultaneously with the amplification oligonucleotide(s) in a reaction or may contact the amplification product subsequent to amplification. A probe may be a nucleic acid that hybridizes to a sequence to be detected (target sequence) including hybridization between DNA/DNA, DNA/RNA, and RNA/RNA strands, or between strands in which one or both strands contain at least one modified nucleotide, nucleoside, nucleobase, and/or base-to-base linkage. Two single strands of sufficient complementarity may hybridize to form a double-stranded structure in which the strands are joined by hydrogen bonds between complementary base pairs (e.g., A with T or U, and G with C) at any point along the hybridized strands. That is, under conditions that promote hybridization between complementary bases, sufficient bonding results in a double-stranded nucleic acid. The rate and extent of hybridization are influenced factors that are well known in the art, which may be predicted by mathematical calculations related to the melting temperature (Tm) for a given hybrid and hybridization solution used, or may be determined empirically by using standard methods (Sambrook, et al., supra Ch. 11). Some embodiments of probe sequences are selected to contain no or a minimum of self-complementarity, whereas other probe embodiments may be partially self-complementary to facilitate detection of probe:target duplexes in a sample without removing unhybridized probe before detection. Examples of partially complementary probes are known, e.g., "molecular beacon" or "molecular switch" probes (e.g., US Pat. Nos. 5,118,801 and 5,312,728, Lizardi et al., US Pat. Nos. 5,925,517 and 6,150,097, Tyagi et al., Glesendorf et al., 1998, Clin. Chem. 44(3):482-6) and "molecular torch" probes (e.g., US Pat. Nos. 6,361,945, Becker et al.). Such probes typically include interacting labels (e.g., luminescent/quencher or fluorophore/quencher pairs) positioned so that a different signal is produced when the probe is self-hybridized compared to when the probe is hybridized to a target nucleic acid.

Detection probes typically have one or more regions of sufficient complementary to hybridize with the target nucleic acid sequence, or its complement, under stringent hybridization conditions (e.g., 60ºC in a solution with a salt concentration of about 0.6-0.9 M). A variety of hybridization conditions are well known (Sambrook et al., id.). Probes of different lengths and base composition may be used, but preferred embodiments include up to 100 bases, preferably from 12 to 50 bases, and more preferably 18 to 35 bases. Probes may be labeled with any known detectable label or reporter group, such as a radioisotope, antigen, fluorescent compound, luminescent moiety (chemiluminescent, electrochemiluminescent, or phosphorescent compound), chromophore, enzyme, enzyme cofactor or substrate, dye, hapten, or ligand for detection of the target sequence associated with the probe (e.g., Sambrook et al., *supra*, Ch. 10; US Pat. No. 6,031,091, Arnold et al.). Preferred embodiments are labeled with an acridinium ester (AE) compound (US Pat. No. 5,185,439, Arnold et al.). Methods of preferentially hybridizing a probe to a target sequence in a sample that may contain other nucleic acids or other biological, organic or inorganic materials, and detecting the signal from the label or reporter group are well known in the art. Preferred embodiments selectively degrade label associated with unhybridized probe and then measure the signal from remaining label associated with hybridized probe (US Pat. No. 5,283,174).

Probes and amplification oligonucleotides may include a sequence that facilitates capture by hybridization with an immobilized oligonucleotide joined to a solid support, by using any known target capture method (e.g., US Pat. No. 6,110,678, Weisburg et al., US Pat. No. 4,486,539, Rankl et al., US Pat. No. 4,751,177, Stabinsky), such as used for nucleic acid purification, which may be performed by using an automated system (e.g., DTS® 1600 Target Capture System, Gen-Probe incorporated, San Diego, CA).

Detection probes may be used in combination with one or more unlabeled helper probes to facilitate binding to target nucleotide sequence, i.e., a probe:target nucleic add duplex more readily forms in the presence of the helper probe than in the absence of the helper probe (US Pat. No. 5,030,557, Hogan et al.). Use of helper probes is particularly preferred when the target nucleic acid (e.g., ssRNA or ssDNA) may contain regions of secondary and tertiary structure even under stringent hybridization conditions because such structures can sterically inhibit or block hybridization of a detection probe to the target nucleic acid. A helper probe contains sequence that hybridizes to a sequence in the target nucleic acid under stringent hybridization conditions but different from the sequence that the detection probe hybridizes. Preferred helper probes are up to 100 bases long, preferably 12 to 50 bases, and more preferably 18 to 35 bases long.

Disclosed herein are kits and diagnostic systems for conducting isothermal amplification and/or for detecting a target sequence. A kit or system may contain, In an amount sufficient for at least one assay, any combination of amplification oligonucleotides, detection probes, helper probes and/or capture probes described herein, and may further include instructions recorded in a a tangible form for use of the components. The components used in an amplification and/or detection process may be provided in a variety of forms, e.g., enzymes, nucleotide triphosphates, probes and/or primers may be provided in lyophilized reagent(s) that, when reconstituted, form a complete mixture of components for use in an assay. A kit or diagnostic systems may contain a reconstitution reagent for reconstituting lyophilized reagent(s). In preferred embodiments for amplifying a target sequence, the enzymes, nucleotide triphosphates and enzyme cofactors are provided as a single lyophilized reagent that, when reconstituted, forms a proper reagent for use in the amplification reaction. Other preferred embodiments provide lyophilized probe reagent(s). Typical packaging materials for such kits and systems include solid matrices (e.g., glass, plastic, paper, foil, micro-particles and the like) that hold detection probes, helper probes and/or amplification oligonucleotides in any of a variety of configurations (e.g., in a vial, microtiter plate well, microarray, and the like). A system, in addition to containing kit components, may further include instrumentation for conducting an assay, e.g. a luminometer for detecting a signal from a labeled probe and/or a magnetic device for separating nucleic add hybridized to a capture probe. Preferred embodiments are illustrated in the following examples, but those skilled in the art will appreciate that other components and conditions in addition to those illustrated may be used In the methods described herein.

### Example 1: Effects of Osmolytes on Isothermal Amplification

Properties of Bst DNA polymerase were examined to determine whether double stranded template nucleic acids is invaded and extended under isothermal conditions. Thermophilic Bst DNA polymerase large fragment has 5' to 3' polymerase activity up to 70 °C but lacks 5' to 3' exonuclease activity. Osmolytes and the base compositions of template nucleic acid and primers were examined for their influence on strand invasion, primer binding, and polymerase extension.

Bst polymerase activity was examined on a 120 nt ssDNA template in which the 3' terminal 10 nt were A/T to provide a "breathing" end. A first inner primer recognized an internal site located 20 nt from the 3'end and was used in the presence or absence of different osmolytes (betaine, proline and trimethylamine N-oxide (TMAO)). Single strand 1 (SS1) was used as a template with the inner primer. The sequence of SS1 (SEQ ID NO:1) was: ATGGTTACGAATTAGTCACTCCGTGGATAGAGTCGCTAGACAGATAAAGCAAGTAGGTATCAACGGACTGAGGG GCAGCACACTACAAGCTTAGAAGATAGAGAGGGATTAAAAAAAAAA. The sequence of the inner primer was CTAAGCTTGTAGTGTGCTGC (SEQ ID NO:2). The Bst reaction mixture (50 µl) contained 1 µM of primer, 250 µM each dNTP, 20 mM Tris-HCL (pH 8.8), 10 mM KCl,10 mM (NH₄)₂SO₄,2 2 mM MgSO₄, 0.1% Triton X-100 and 8 Units Bst DNA polymerase large fragment, which was incubated 1 hr at 60 ºC. Amplification products were detected using a standard hybridization protection assay (HPA) using an AE-labeled detection probe (substantially as described in US Pat. Nos. 5,283,174 and 5,639,604). The detection probe sequence was GCAAGTAGGTTATCAACGGACTGAGG (SEQ ID NO:3, labeled with AE via a linker between nt 11 and 12). Briefly, detection included a hybridization step in which an excess of AE-labeled probe was mixed with the reaction and hybridized to the amplified target sequence, followed by a selection step in which an alkaline reagent was added to hydrolyze AE label associated with unhybridized probe making it non-chemiluminescent, and then chemiluminescence from AE of the probe:target hybrid was measured by using a luminometer (expressed in relative light units or "(RLU"). In these tests, the AE-labeled probe hybridized to the SS2 strand (sequence complementary to the SS1 strand). Here, HPA was carried out in a denaturing format, but a non-denaturing format was used in other tests (e.g., Example 2). In denaturing HPA, samples are heated 5 min at 95 ºC, cooled to about 70 ºC and AE-labeled probe was added (10 fmol), whereas non-denaturing HPA omits the separate heating and cooling steps.

Replicate tests were conducted without an osmolyte or with 1M betaine, 0.5M or 1M proline, or 0.25M or 0.5M TMAO, using different amounts of inner primer (10⁷, 10⁸ and 10⁹ copies/ml). Results (RLU detected) are shown in Tables 1A, 1B and 1C.

**TABLE 1A: Tests Without Osmolyte or With Betaine**

| **Inner Primer (copies/ml) + Osmolyte** | **10⁹ + None** | **10⁸ + None** | **10⁷ + None** | **10⁹ + 1M Betaine** | **10⁸ + 1M Betaine** | **10⁷ + 1M Betaine** |
|---|---|---|---|---|---|---|
| **Test 1** | 52,342 | 3,139 | 1,105 | 454,409 | 488,701 | 485,295 |
| **Test 2** | 57,924 | 3,214 | 1,006 | 462,122 | 519,908 | 509,285 |

**TABLE 1B: Tests With Proline**

| **Inner Primer (copies/ml) + Osmolyte** | **10⁹ + 1M proline** | **10⁸ + 1M proline** | **10⁷ + 1M proline** | **10⁹ + 0.5M proline** | **10⁸ + 0.5M proline** | **10⁷ + 0.5M proline** |
|---|---|---|---|---|---|---|
| **Test 1** | 1044 | 664 | 530 | 8826 | 795 | 672 |
| **Test 2** | 1202 | 609 | 519 | 8779 | 743 | 694 |

**TABLE 1C: Tests With TMOA**

| **Inner Primer (copies/ml) + Osmolyte** | **10⁹ + 0.5M TMOA** | **10⁸ + 0.5M TMOA** | **10⁷ + 0.5M TMOA** | **10⁹ + 0.25M TMOA** | **10⁸ + 0.25M TMOA** | **10⁷ + 0.25M TMOA** |
|---|---|---|---|---|---|---|
| **Test 1** | 20095 | 2602 | 890 | 546,383 | 139,958 | 1056 |
| **Test 2** | 23524 | 2593 | 959 | 590,229 | 150,308 | 3101 |

When 1 M betaine was present and 10⁷ copies of primer were used, an average 497,290 RLU was detected compared to an average 1055 RLU for reactions without betaine. Using 10⁷ copies of primer, one extension was expected to yield 1583 RLU and, thus an estimated 314-fold amplification was achieved with betaine present, which is a minimal estimate because all available AE probe (10 fmol) was consumed in the reaction. When 0.25 M TMAO was present, the reactions also demonstrated catalytic turnover, producing an average 145,143 RLU using 10⁸ copies of primer, i.e., 9-fold amplification compared to an expected value of 15,830 RLU for one extension at this primer level. When proline was present, no substantial stimulation of amplification was seen.

### Example 2: Effects of an AT-Rich Primer on Isothermal Amplification

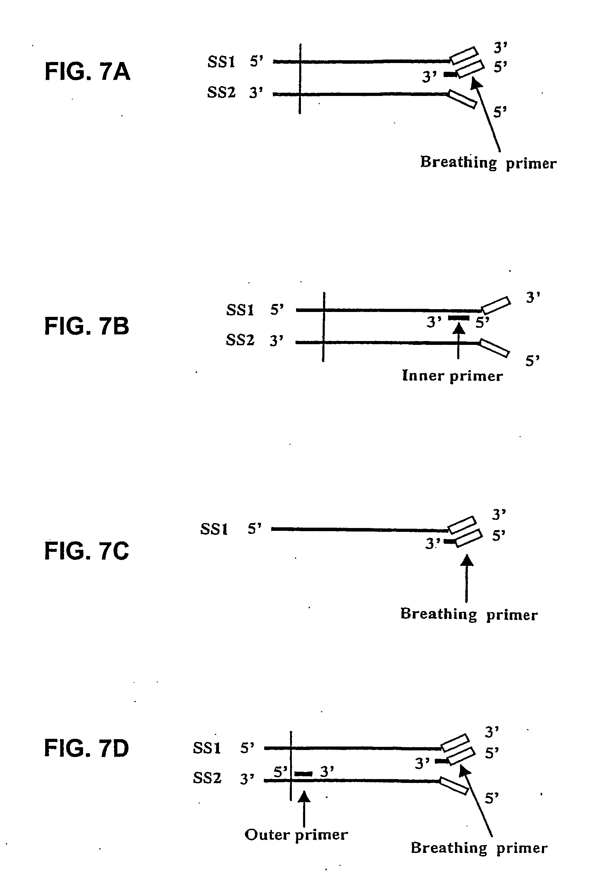

Bst polymerase activity was examined on a ssDNA and dsDNA (120 nt) template having one AT-rich end by using an AT rich primer (referred to as a "breathing primer") to determine whether catalytic turnover occurs in the absence of an osmolyte. The ssDNA template was SS1 oligonucleotide (SEQ ID NO:1), and the dsDNA template was the SS1 oligonucleotide hybridized to a complementary oligonucleotide (SS2, SEQ ID NO:4) having the sequence TTTTTTTTTAATCCCTCTCTATCTTCTAAGCTTGTAGTGTGCTGCCCCTCAGTCCGTTGATACCTACTTGCTTTAT CTGTCTAGCGACTCTATCCACGGAGTGACTAATTCGTAACCAT.
Three primers were tested: (1) a first primer, the breathing primer (TTTTTTTTTTAATCCCTCTC, SEQ ID NO:5), that was complementary to the 3' terminal 20 nt of the SS1 template; (2) a second primer, referred to as an "inner primer," that was complementary to an internal site located 20 nt from the 3'terminus of the SS1 template (SEQ ID NO:2); and (3) a third oligonucleotide (ATAGAGTCGCTAGACAGA, SEQ ID NO:6), referred to as an "outer primer," that was complementary to an internal sequence located 20 nt from the 3' terminus of the SS2 strand. The reactants were tested in mixtures containing different amounts of template (10² to 10¹⁰ copies/ml) in the following combinations: (1) dsDNA template and breathing primer (illustrated in FIG. 7A); (2) dsDNA template and inner primer (illustrated in FIG. 7B); (3) ssDNA template and breathing primer (illustrated in FIG. 7C); and (4) dsDNA template, outer primer, and breathing primer (illustrated in FIG. 7D). In tests involving the dsDNA template, the template was prepared by hybridizing the SS1 and SS2 strands together (incubated at 95 ºC for 5 min, then on ice for 10 min in a 50 mM NaCl solution). In these tests, the Bst reaction mixture (50 µl) contained 1 µM primer (50 pmoles), 250 µM of each dNTP, 20 mM Tris-HCL (pH 8.8),10 mM KCl,10 mM (NH₄)₂SO₄, 2 mM MgSO₄, 0.1% Triton X-100 and 8 Units Bst DNA polymerase large fragment, which was incubated at 60 ºc for 1 hr. Results of the tests are shown in Table 2. Denaturing HPA detection methods (indicated by an asterisk in Table 2) and non-denaturing HPA detection methods, as described in Example 1, were used to detect the SS2 strand.

**TABLE 2: RLU Detected for Tests Using Different Template and Primer(s) Combinations**

| **Template + Primer(s)** | **10¹⁰ copies/ ml** | **10⁹ copies/ ml** | **10⁸ copies/ ml** | **10⁷ copies/ ml** | **10⁶ copies/ ml** | **10⁵ copies/ ml** | **10⁴ copies/ ml** | **10³ copies ml** | **10² copies/ ml** |
|---|---|---|---|---|---|---|---|---|---|
| **dsRNA+ Breathing primer** | 387,078 | 346,504 | 335,690 | 404,638 | 413,111 | 10,347 | 1369 | 1137 | 1011 |
| **dsDNA + Breathing Primer*** | 419,609 | 444,376 | 445,968 | 519,129 | 183,245 | 4533 | 2970 | 771 | 796 |
| **dsDNA+ Breathing Primer** | 775,187 | 702,632 | 597,991 | 233,841 | 202,291 | 1405 | 1419 | 1516 | 994 |
| **ssDNA + Breathing Primer*** | 852,266 | 762,911 | 359,575 | 774,705 | 117,778 | 6219 | 1436 | 1329 | 1212 |
| **dsDNA + Breathing & Outer Primers** | 1249 | 2042 | 2661 | 1632 | 2649 | 1087 | 1034 | 1372 | 699 |
| **dsDNA+ Breathing & Outer Primers*** | 600,597 | 844,671 | 683,977 | 229,363 | 881,580 | 34,334 | 41,129 | 4752 | 2678 |
| **dsDNA + Inner Primer** | | | 1469 | 959 | 728 | 796 | 798 | 679 | 612 |
| **dsDNA + Inner Primer*** | | | 4017 | 893 | 617 | 633 | 572 | 613 | 598 |

For amplification of the dsDNA template with breathing primer alone and Inner primer alone, greater amplification was detected at the 10⁶ template copy level using the breathing primer (183,245 RLU) than using the inner primer (617 RLU), compared to the expected RLU for one amplification (158 RLU at the 10⁶copy level). Thus, approximately 1157-fold amplification was seen with the breathing primer, indicating more catalytic turnover occurred by using the breathing primer than occurred by using the inner primer. Amplification of the ssDNA template was approximately 743-fold using the breathing primer at 10⁶ copies (117,778 RLU). Combining the outer primer with the breathing primer provided 2.5 X 10⁴-fold amplification of the dsDNA template at 10⁴ copies/ml of template, indicating enhanced amplification when the two primers were used. Thus, in the absence of an osmolyte, an AT-rich primer which recognizes an AT-rich end of either the ssDNA or dsDNA template stimulated isothermal amplification.

### Example 3: Effects of Primer Concentration and Osmolyte on Isothermal Amplification

Amplification of the 120 nt template used in Examples 1 and 2 by using an inner primer and outer primer was determined at two primer concentrations, in the presence or absence of 1 M betaine. Template dsDNA, outer primer, inner primer and amplification conditions were as described in Example 2 with primer concentrations of 0.1 µM or 1 µM. Amplification products were detected by using denaturing HPA (see Example 1). Replicate tests with different amounts of the template (copies/ml) and 5 pmoles or 50 pmoles of primer were performed. The results (RLU detected) are reported in Table 3, which includes a control test in which template was amplified with the breathing primer as described in Example 2.

**TABLE 3: RLU Detected Using Different Primer, Template, and Osmolyte Combinations**

| **Copies/ml of dsDNA + Primer(s) + Osmolyte** | **5 pmol primer Test1** | **5 pmol primer Test 2** | **50 pmol primer Test 1** | **50 pmol primer Test 2** |
|---|---|---|---|---|
| **10⁵ Template+ Outer & Inner Primers +** 1 **M betaine** | 69,412 | 2805 | 1,420,857 | Not determined |
| **10⁹ Template+ Outer & Inner Primers + 1M betaine** | 48,637 | 37,390 | 794,542 | 1,521,518 |
| **10⁷ Template+ Breathing primer + No osmolyte** | 11891 | 44,811 | 303,542 | 480,570 |
| **10⁵ Template + Outer & Inner Primers + No osmolyte** | 1459 | 1455 | 1512 | 1741 |

At 10⁵ copies/ml of template, 89738-fold amplification was detected in the presence of betaine and insubstantial amplification was detected in reactions without betaine (e.g., 1.4 x10⁶ RLU with betaine compared to 1.6 x 10² RLU without betaine). This is a minimum estimate of increased amplification because all available detection probe was consumed in the reaction. Greater amplification was detected when 50 pmol primer was used, compared to reactions that used 5 pmol primer.

Similar tests were performed in triplicate using reactions that contained 10⁵ copies/ml of dsDNA template and 50 pmoles of outer and inner primers, with or without 1 M betaine. Triplicate assays performed in the presence of osmolyte resulted in a mean of 1.4 x 10⁶ RLU compared to a mean of 8.45 x 10² RLU for reactions without osmolye, i.e., at least an 88,455-fold amplification increase in the presence of betaine.

### Example 4: Determination of Template Copies Required for Isothermal Amplification

Amplification levels of 10⁶,10⁵ and 10⁴ copies/ml of a dsDNA template by using a combination of the inner primer and outer primer were determined in the presence of 1 M betaine, as described in Example 3, but using a different dsDNA template. The dsDNA template in these tests included two AT-rich termini in which the dsDNA was composed of hybridized SS1 and SS2 oligonucleotides as shown below.
SS1 (SEQ ID NO:7):
SS2 (SE4 ID N0:8):
The reaction conditions were as described in Example 2, with or without betaine, and amplification products were detected by denaturing HPA as described In Example 1. In these tests, a single extension was expected to yield less than 158 RLU, and any higher signal indicates catalytic turnover. The positive control contained 10¹⁰ copies/ml ssDNA template but no osmolyte, and the negative controls contained no template DNA or osmolye, or dsDNA template and primers but no osmolyte. At 10⁸ copies/ml of dsDNA template, two of three replicates were positive, at 10⁵ copies/ml of dsDNA template, all three replicates were positive, and at 10⁴ copies/ml of dsDNA template one of three replicates was positive. Thus, reactions containing 10⁴ copies/m template are appropriate for detecting positive results. For 10⁴ copies/ml of dsDNA template, the presence of betaine enhanced amplification 4.4 X 10⁵-fold (691,922 RLU were detected for a reaction with betaine compared to 929 RLU detected in the reaction containing 10⁶ copies/ml of dsDNA template without betaine). The results are shown in Table 4 as the RLU of triplicate tests.

**TABLE 4: Amplification of Templates With and Without Osmolye**

| **Template, Primer(s), Osmolyte** | **10⁶ copies/ml Template** | **10⁵ copies/ml Template** | **10⁴ copies/ml Template** |
|---|---|---|---|
| **dsDNA template + Outer and Inner Primers 1 M Betaine** | 578,660 | 211,440 | 691,922 |
| | 1046 | 658,026 | 1430 |
| | 752,328 | 708,179 | 1409 |
| **ssDNA template+ Inner primer No osmolyte,** | 182,011 | (not tested) | (not tested) |
| | 223,550 | | |
| | 611,348 | | |
| **Negative Control No template or primers No osmolyte** | 823 | (not tested) | (not tested) |
| | 869 | | |
| | 1965 | | |
| **Negative Control dsDNA template + Outer and Inner Primers No osmolyte** | 983 | (not tested) | (not tested) |
| | 940 | | |
| | 866 | | |

### Example 5: Effects of Reaction Conditions on Amplification Positivity

Additional embodiments of isothermal amplification methods were tested in reactions containing 10⁴ copies/ml of the dsDNA template and inner and outer primers, with or without 1 M betaine, substantially as described in Example 4 using conditions similar to those described in Example 2 with the exceptions described herein. Amplification products were detected by using denaturing HPA (described In Example 1). Assays were performed using 200 pmol of primers, in amplification reactions that included variables described in Tables 5A and 5B (reactions that used 10X Bst enzyme concentration, or contained 250 mM KCl,10 mM MgSO₄, or 50 µg of bovine serum albumin (BSA) compared to standard conditions). Positive controls were standard reactions that contained 10⁹ or 10¹⁰ copies/ml of ssDNA template and only inner primer. Negative controls contained no template or template plus primers without osmolyte. The RLU results of triplicate tests for each reaction condition are shown in Tables 5A and 5B.

**TABLE 5A**

| **200 pmol primers** | **10 X *Bst* enzyme** | **250 mM KCI** | **Positive control** | **Negative control** | **Negative control** |
|---|---|---|---|---|---|
| **1M betaine** | **1M betaine** | **1M betaine** | **No osmolyte** | **No osmolyte** | **No template** |
| **dsDNA + outer & inner primers** | **dsDNA + outer & Inner primers** | **dsDNA + outer & inner primers** | **ssDNA (10¹⁰) Inner primer** | **DsDNA + Outer & inner primers** | |
| 758,007 | 819 | 1276 | 165,610 | 867 | 1078 |
| 1511 | 667 | 957 | 173,187 | 817 | 859 |
| 736,938 | 854 | 1078 | 176,956 | 1738 | 800 |

**TABLE 5B**

| **200 pmol primers** | **200 pmol primers** | **200 pmol primers** | **Positive control** | **Negative control** | **Negative control** |
|---|---|---|---|---|---|
| **1 M betaine** | **50 µg BSA 1M betaine** | **10 mMMgSO₄ 1 M betaine** | **No osmolyte** | **No osmolyte** | **No template** |
| **dsDNA +outer & inner primers** | **dsDNA + outer & inner primers** | **dsDNA + outer & inner primers** | **ssDNA (10⁹) inner primer** | **DsDNA + Outer & inner primers** | |
| 797,576 | 931 | 572,557 | 85,771 | 1250 | 1595 |
| 1709 | 808,220 | 47,334 | 84,453 | 1129 | 1415 |
| 1126 | 762,344 | 841 | 84,810 | 1720 | 1390 |

Use of 200 pmol of outer and inner primers instead of 50 pmol (Example 2) increased the number of positive results (from 1 of 3 to 2 of 3), and addition of 50 µg BSA increased the positive results (2 of 3), but increasing the enzyme, KCI or MgSO₄ concentrations did not increase positives.

### Example 6: Effects of Temperature on Amplification Positivity

Similar isothermal amplifications were performed to determine the effects of temperature on amplification, using reactions that contained 10⁴ copies/ml of dsDNA, Inner and outer primers (200 pmol), and 1 M betaine or no osmolyte. Template dsDNA was as described in Example 4, amplified using conditions as described in Examples 2 and 5, using 200 pmoles of primers and 50 µg BSA in reactions incubated 1 hr at 55, 65, or 70 ºC, and amplification products were detected by using denaturing HPA (described in Example 1 A positive control contained 10⁹ copies/ml of ssDNA template and inner primer, and a negative control contained no template. Table 6 shows the results (RLU of three test sets) for amplification reactions performed at 55 ºC, 65 ºC and 70 ºC.

**TABLE 6A**

| **55 ºC** | **65 ºC** | **70 ºC** | **Positive control** | **Negative control** | **Negative control** |
|---|---|---|---|---|---|
| **50 µg BSA** | **50 µg BSA** | **50 µg BSA** | | | |
| **1M betaine** | **1M betaine** | **1M betaine** | **No osmolyte** | **No osmolyte** | **No template** |
| **dsDNA + outer & inner primers** | **dsDNA + outer & Inner primers** | **dsDNA + outer & inner primers** | **ssDNA + inner primer** | **DsDNA + outer & inner primers** | |
| Set 1: | Set 1: | Set 1: | Set 1: | Set 1: | Set 1: |
| 2520 | 629,227 | 3164 | 28,397 | 1147 | 964 |
| 384,970 | 595,352 | 2416 | 27,044 | 1052 | 948 |
| 1480 | 5,799 | 1901 | 26,664 | (no triplicate) | (no triplicate) |
| | | | | | |
| Set 2: | Set 2: | Set 2: | Set 2: | Set 2: | Set 2: |
| 1750 | 1325 | 1676 | 56,117 | 997 | 803 |
| 612,730 | 650,942 | 1310 | 52,080 | 815 | 738 |
| 1283 | 1077 | 1368 | 51,833 | 1150 | 3171 |
| | | | | | |
| Set 3: | Set 3: | Set 3: | Set 3: | Set 3: | Set 3: |
| 643,140 | 672,353 | 1237 | 25374 | 1933 | 1147 |
| 650,253 | 581,170 | 1002 | 23876 | 1441 | 1102 |
| 845 | 1034 | 1133 | 23775 | 1503 | 1132 |

At reaction temperatures of 55 ºC, 65 ºC and 70 ºC, results were positive for 44% of the tests (four of nine reactions), 66% of the tests (six of nine reactions) and 0% of the tests (zero of nine reactions), respectively. Thus, about 65ºC appears to the optimum reaction temperature.

### Example 7: Effects of Osmolyte on Strand Invasion

A standard melting temperature (Tₘ) study (i.e., absorbance at 260 nm as a function of temperature) was conducted on the dsDNA template described in Example 2 to determine how the presence of osmolyte (1M betaine) influences Tₘ. The presence of osmolyte decreased the Tₘ of the dsDNA template from 80.0 ºC (without betaine) to 78.7 ºC (with betaine), i.e., decreased only 1.3 ºC, indicating that the osmolyte did not significantly destabilize the dsDNA. The system temperature (65 ºC) did not significantly destabilize the dsDNA because it was below the Tₘ of the dsDNA template (80 °C).

The influence of the presence of osmolyte (1M betaine) on strand invasion of the dsDNA template by the breathing primer without polymerase were examined using a strand invasion assay reaction that contained BST buffer (20mM Tris, pH 7.5, 10mM KCl,10 mM (NH₄)₂SO₄, 2mM MgCl₂. 0.1% Triton),1M betaine, and AE-labeled probe of sequence TTTTTTTTTTAATCCCTCTCTATCTTCTAAGCTTG (SEQ ID NO:9, labeled with AE by a linker between nt 21 and nt 22), and a gel-purified dsDNA template made up of the synthetic strands shown below: In the reactions, 1 pmole of template was hybridized to 0, 0.1, 0.25, 0.5 and 1.0 pmole of AE-labeled probe at 65ºC for 30 min in BST buffer, then 10 µl of the reaction mixture was removed, diluted to 1.0 ml with BST buffer, and 80 µl of the diluted mixture was used in an adduct protection assay (APA) (substantially as described in US Pat. No. 5,731,148) in which the mixture first was mixed with 0.2 ml of 14 mM sodium sulfite and 42 mM borate, pH 8.8, and 12 sec later with 0.2 ml of 0.12% H₂O₂ and 1.5N NaOH. Assay products were detected (2 sec) as RLU in a luminometer. The RLU results shown in Table 7 indicate that the presence of osmolyte did not significantly influence strand invasion in the absence of polymerase.

**TABLE 7: Strand Invasion Assay**

| **Pmol primer** | **Background RLU** | **Primer RLU** | **Net** | **% Invasion** |
|---|---|---|---|---|
| **No betaine** | | | | |
| 0.1 | 145 | 204 | 59 | 0.11 |
| 0.25 | 228 | 262 | 34 | 0.07 |
| 0.5 | 359 | 528 | 169 | 0.33 |
| 1.0 | 533 | 630 | 97 | 0.19 |

| **1M betaine** | | | | |
|---|---|---|---|---|
| 0.1 | 123 | 149 | 26 | 0.06 |
| 0.25 | 164 | 232 | 68 | 0.16 |
| 0.5 | 231 | 299 | 68 | 0.16 |
| 1.0 | 402 | 648 | 246 | 0.57 |

A similar test was performed with the dsDNA template and outer primer as described in Example 2, which yielded results consistent with the above conclusion, i.e., osmolyte did not significantly effect strand invasion when polymerase was absent. These observations combined with Tₘ measurements show that an osmolyte did not significantly destabilize the template and instead may enhance strand displacement by acting on or with the polymerase enzyme.

### Example 8: Effects of Template Base Composition on Isothermal Amplification

To determine whether base composition affected the probability of invasion and extension, two dsDNA templates were compared: one having two AT-rich termini and one having two GC-rich termini (45% GC). Amplification reactions were performed using conditions as described in Example 6 with the two dsDNA templates that differed at their termini (GC-rich or AT-rich termini). The dsDNA template having GC-rich termini was made up of synthetic strands having the following nucleotide sequences: and The dsDNA template having AT-rich termini was comprised of strands having the following nucleotide sequences: and The test reactions included 10⁴ copies/ml of template, 200 pmol of each primer (listed In Table 8A), 50 pg of BSA, 1 M betaine, and were incubated 1 hr at 65 ºC. The controls included no betaine and used inner primer with ssDNA template at 10⁹ copies/ml (positive control), or dsDNA plus inner and outer primers (betain negative control), or no template or primer (negative control). Table 8A shows the RLU results.

**TABLE 8A**

| **Test reaction With betaine** | **Test reaction with betaine** | **Test reaction with betaine** | **Positive control** | **Negative control (no betaine) dsDNA + Outer +inner primers** | **Negative control (no template)** |
|---|---|---|---|---|---|
| **DsDNA + Outer +inner primers** | **dsDNA + Outer +inner primers** | **dsDNA + Outer +Inner primers** | **ssDNA + Inner primer** | | |
| 1887 | 1543 | 1213 | 64,847 | 1251 | 981 |
| 1643 | 1504 | 88,679 | 63,141 | 1129 | 2841 |
| 1646 | 1142 | 630,152 | 62,397 | 1105 | 2207 |

In reactions that used the template with GC-rich ends, 22% of the reactions showed positivity (2 of 9 reactions were positive), whereas reactions that used the template with AT-rich ends showed 66% positivity (6 of 9 reactions were positive), suggesting that the template's terminal base composition influences strand invasion and primer extension.

Amplification reactions were performed on a different day under the same conditions as for the reactions reported in Table 8A, except that only inner primer or only outer primer was added to the reaction. Results of these reactions are reported in Table 8B.

**TABLE 8B**

| **Test reaction with Betaine** | **Test reaction with betaine** | **Test reaction with ' betaine** | **Positive control** | **Negative control (No template)** |
|---|---|---|---|---|
| **dsDNA + Inner + outer primers** | **dsDNA + Inner primer** | **dsDNA + Outer primer** | **ssDNA + Inner primer** | |
| 3350 | 1179 | 2424 | 88,601 | 1341 |
| 401,593 | 918 | 1107 | 85,363 | 1228 |
| 2187 | 647 | 5659 | 84,648 | 1053 |
| 1971 | 2159 | 1018 | | |
| 1647 | 656 | 1003 | | |
| 893,738 | 801 | 1210 | | |
| 1323 | 827 | 1944 | | |
| 1040 | 884 | 1851 | | |
| 1713 | 1661 | 1802 | | |

Positivity for the dsDNA template amplified with the inner and outer primers was reproduced (22% positivity, 2 of 9 reactions), but no positive results were seen when, only an inner or outer primer was used to amplify 10⁴ copies/ml of template, suggesting maximal activity requires both primers.

### Example 9: Effects of Number of Strands in Template on Isothermal Amplification

To determine whether the number of strands in template DNA affected the probability of strand invasion and extension, amplification of a ssDNA template was compared to amplification of a dsDNA template. Amplification reactions were performed using conditions as described in Example 6 (1 hr at 65 ºC) with template having GC-rich termini at 10⁴ copies/ml. The positive control used inner primer with ssDNA template at 10⁹ copies/ml. RLU results of the isothermal amplification reactions are shown in Table 9A.

**TABLE 9A**

| **Test reaction with Betaine** | **Test reaction with Betaine** | **Test reaction with Betaine** | **positive control (no betaine)** | **Negative control (no betaine)** | **Negative control (No template)** |
|---|---|---|---|---|---|
| **ssDNA + Inner + outer primers** | **ssDNA + inner+ outer primers** | **SsDNA + Inner + Outer primers** | **ssDNA + Inner primer** | **ssDNA + Inner + outer primers** | |
| 126,970 | 2652 | 1175 | 50,524 | 4292 | 1039 |
| 2224 | 1753 | 234,151 | 75,353 | 1353 | 1235 |
| 1620 | 728,475 | 795,393 | 34,426 | 1272 | 885 |

In these reactions, the ssDNA template resulted in 44% positivity (4 of 9 were positive), whereas the dsDNA template resulted in 22% positivity (2 of 9 were positive; see Example 8). These results suggest primer location at the end of a template can increase primer hybridization and polymerase extension.

Amplification reactions were performed on a different day under the same conditions as for those reported in Table 9A, except that only inner primer and only outer primer was added to the reaction. Results of these reactions are reported in Table 9B.

**TABLE 9B**

| **Test reaction with Betaine** | **Test reaction with Betaine** | **Test reaction with Betaine** | **Positive control (no betaine)** | **Negative control** |
|---|---|---|---|---|
| **ssDNA + Inner + Outer primers** | **ssDNA + Inner primer** | **ssDNA + Outer primer** | **ssDNA + inner primer** | **(no template)** |
| 2505 | 2332 | 1193 | 86,161 | 789 |
| 2181 | 1978 | 1021 | 88,961 | 1105 |
| 404,911 | 3745 | 1735 | 88,004 | 4314 |
| 1584 | 2671 | 1025 | | |
| 1411 | 1867 | 2516 | | |
| 359,918 | 1326 | 1279 | | |
| 1340 | 1131 | 1016 | | |
| 573,538 | 1145 | 1899 | | |
| 1211 | 1103 | 806 | | |

Thirty-three percent (33%) positivity was observed for reactions with inner and outer primers and ssDNA (3 of 9 were positive), which was comparable to the 44% positivity observed in Table 9A. A single primer with betaine did not provide positivity at 10⁴ template, indicating that two primers are needed in the reaction.

### Example 10: Primer Hybridization Locations in Templates Used In Isothermal Amplification

Primer accessibility to dsDNA template and it influence on isothermal amplification were examined by comparing amplification of two dsDNA templates: a 120 nt template to which primers hybridized 20 bases from each terminus and a 81 nt template to which primers hybridized at each terminus. The 120 nt dsDNA template was made up of the following two strands: and The 81 nt dsDNA template was made up of the following two strands: and Using reaction conditions as described in Example 8, amplification of the 81 nt ssDNA template was compared to the 81 nt dsDNA template (10⁴ copies/ml per reaction) in the presence of 1 M betaine. The positive control without betaine used 10⁹ copies/ml per reaction of the ssDNA target, and the negative controls without betaine used 10⁴ copies/ml of the ssDNA target or no template. Table 10 shows the results.

**TABLE 10**

| **Test reaction with Betaine** | **Test reaction with Betaine** | **Positive control** | **Negative control** | **Negative control (No template)** |
|---|---|---|---|---|
| **81 nt ssDNA + inner+outer primers** | **81 nt dsDNA + inner+outer primers** | **ssDNA + inner primer** | **ssDNA + inner+outer primers** | |
| 440,170 | 1620 | 61,859 | 1016 | 1404 |
| 2984 | 635,843 | 60,606 | 1094 | 1061 |
| 2481 | 682,659 | 61,418 | 917 | 1016 |
| 530,852, | 653,154 | | | |
| 1537 | 1391 | | | |
| 1723 | 1280 | | | |
| 1729 | 1153 | | | |
| 654,321 | 1455 | | | |
| 1237 | 1961 | | | |

The 81 nt ssDNA template and dsDNA template each resulted in 33% positivity (3 of 9 were positive replicates), which were similar to the results observed for the 120 nt ssDNA GC-rich template in Example 9 (44% positivity), for which there was higher positivity than for amplification of the 120 nt template having internal primer hybridization sites. Thus, primers hybridizing at the template termini increased primer hybridization and polymerase extension. Greater amplification was observed for a 120 nt dsDNA template with AT-rich termini (66% positivity, Example 6), suggesting that destabilization of a template terminus increases strand invasion and amplification.

### Example 11: Isothermal Amplification of Template with AT-Rich Termini

The influence of the number of strands in a template having AT-rich termini on isothermal amplification was determined by comparing amplifications of ssDNA and dsDNA templates (10⁴ copies/ml per reaction) using reaction conditions as described in Example 8. The results are shown in Table 11, in which the test reactions contained 1M betaine (columns 2 to 4), the positive control (column 5) included 10⁹ copies/ml of the ssDNA template and the inner primer but no betaine, and the negative control contained no template or betaine (column 6).

**TABLE 11**

| **Template** | **Inner+outer primers** | **Inner primer** | **outer primer** | **Positive control** | **Negative Control** |
|---|---|---|---|---|---|
| **dsDNA** | 1655 | 2362 | 1254 | 94,954 | 950 |
| | 802,078 | 1635 | 1156 | 91,916 | 944 |
| | 858,743 | 1857 | 1170 | 72,912 | 877 |
| | 1372 | 1764 | 1056 | | |
| | 2756 | 1928 | 3312 | | |
| | 673,386 | 3291 | 925 | | |
| | 789,742 | 1589 | 1128 | | |
| | 875,665 | 1215 | 2845 | | |
| | 1117 | 1523 | 1024 | | |
| **ssDNA** | 862,890 | 2033 | 988 | 107,801 | 723 |
| | 835,005 | 1990 | 1017 | 107,553 | 282 |
| | 96,873 | 1669 | 949 | 102,091 | 1102 |
| | 936,601 | 4819 | 860 | | |
| | 1373 | 1367 | 843 | | |
| | 1310 | 1391 | 836 | | |
| | 864,228 | 2328 | 1070 | | |
| | 3334 | 1390 | 935 | | |
| | 838 | 1010 | 904 | | |

Fifty-five percent (55%) positivity was observed (5 of 9 positive) when the outer and inner primers were used to amplify dsDNA template, consistent with results of Example 6 (66% positivity). Amplification of ssDNA using the two primers was consistently seen (55% positivity in Table 11, and 44% positivity observed in a separate test). Single primer reactions did not result in positivity, Indicating exponential amplification required two primers. Positivity of dsDNA with two AT-rich termini was greater than with a dsDNA template without AT-rich termini, suggesting that the template's base composition at its terminus influences invasion, primer hybridization and polymerase extension.

### Example 12: Isothermal Amplification of dsDNA Template Having One AT-Rich Terminus

The influence of having one AT-rich terminus in a dsDNA template on amplification positivity was determined by comparing amplification of a dsDNA template having one AT-rich end. The dsDNA template (10⁴ copies/ml per reaction) was as described in Example 2, used in reaction conditions as described in Example 8. The positive control used 10⁹ copies/ml of the ssDNA template with only inner primer and no betain, whereas the negative control used 10⁴ copies/ml of dsDNA template with inner and outer primers without betaine. Results are shown in Table 12.

**TABLE 12**

| **Test reaction with Betaine** | **Test reaction with Betaine** | **Test reaction with Betaine** | **Positive control** | **Negative control** |
|---|---|---|---|---|
| **DsDNA + inner+Outer primers** | **DsDNA + Inner Primer** | **DsDNA + Outer primer** | | |
| 2768 | 1459 | 1234 | 85,292 | 1267 |
| 16,880 | 5856 | 1904 | 86,320 | 1017 |
| 386,743 | 1679 | 1772 | 84,458 | 1265 |
| 1983 | 1636 | 1051 | | |
| 604,216 | 1547 | 968 | | |
| 489,915 | 1430 | 2196 | | |
| 1352 | 1240 | 1051 | | |
| 654,137 | 1712 | 1011 | | |
| 817 | 2162 | 1341 | | |

The dsDNA template having one AT-rich terminus was amplified with 55% positivity (5 of 9 replicates were positive), which is comparable to results obtained with the template having two AT-rich termini (positivity of 66% in Example 6 and 55% in Example 11), suggesting that the primer hybridization site is accessible and influenced by the Bst polymerase's strand displacement activity, and a second AT-rich terminus is not necessary for effective amplification.

## Claims

1. An isothermal nucleic acid amplification method comprising:
a) providing a reaction mixture that includes
i) a nucleic acid template strand;
ii) extension nucleotides;
iii) a first oligonucleotide primer that contains a 5'-terminal AT-rich sequence X that is not complementary to or consisting of a sequence in the nucleic acid template strand and a 3'-terminal sequence Z that is complementary to a sequence in the nucleic acid template strand, wherein the AT-rich sequence X is about 10-40 nt in length and is made up of 65-100% A and T residues;
iv) a second oligonucleotide primer consisting of a sequence contained in the nucleic acid template strand; and
v) a nucleic acid polymerase having strand displacement activity;
b) hybridizing sequence Z of the first oligonucleotide primer to a complementary sequence in the nucleic acid template strand, wherein, if the nucleic acid template strand is one strand of a double-stranded nucleic acid, then the double-stranded nucleic acid is chemically or physically denatured before hybridizing the first oligonucleotide primer to the nucleic acid template strand;
c) synthetically extending the first oligonucleotide primer from the 3' terminus of sequence Z by nucleic acid polymerization to make sequence Y that is complementary to at least part of the nucleic acid template strand, thereby forming a first strand of a double-stranded nucleic acid that is isothermally amplified;
d) at least partially separating sequence Y from the nucleic acid template strand;
e) hybridizing the second oligonucleotide primer to a complementary sequence contained in sequence Y;
f) synthetically extending the 3' terminus of the second oligonucleotide primer by nucleic acid polymerization, thereby forming a second strand of the double-stranded nucleic acid that is isothermally amplified, in which the second strand contains an AT-rich sequence complementary to sequence X of the first oligonucleotide primer, thereby forming an AT-rich region of the double-stranded nucleic acid that is isothermally amplified;
g) hybridizing the first oligonucleotide primer to the second strand of the double-stranded nucleic acid that is isothermally amplified under conditions where the AT-rich region of the double-stranded nucleic acid is partially opened to make the second strand accessible to the first oligonucleotide primer; and
h) polymerizing an extension product of the first oligonucleotide primer hybridized to the second strand by using the nucleic acid polymerase having strand displacement activity, thereby displacing the first strand of the double-stranded nucleic acid and performing at least one amplification cycle under isothermal conditions on the double-stranded nucleic acid that is isothermally amplified.

2. The method of claim 1, wherein the amplification cycle under isothermal conditions further includes hybridizing the second oligonucleotide primer to the first strand that was displaced by polymerizing to form the extension product of the first oligonucleotide primer, and extending the 3' terminus of the second oligonucleotide primer by nucleic acid polymerization using the first strand as a template.

3. The method of claim 1 or 2, wherein the nucleic acid template strand is ssRNA, and wherein the reaction mixture further includes an enzyme with reverse transcriptase (RT) activity and a means for cleaving RNA, whereby the RT activity synthetically extends the first oligonucleotide primer from the 3' terminus of sequence Z to make sequence Y in the first strand and the means for cleaving RNA degrades the ssRNA template strand during or after synthesis of the first strand.

4. The method of claim 1 or 2, wherein the nucleic acid template strand is ssDNA, and wherein the method further includes a step of chemically or physically denaturing the nucleic acid template strand from the first strand made by synthetically extending the first oligonucleotide primer from the 3' terminus of sequence Z by nucleic acid polymerization to make sequence Y that is complementary to at least part of the nucleic acid template strand.

5. The method of claim 1 or 2, wherein the nucleic acid template strand is ssDNA, and wherein the method further comprises:
in the providing step, providing in the reaction mixture a third oligonucleotide that includes sequence T that hybridizes to a sequence in the nucleic acid template strand located 3' of the sequence to which sequence Z hybridizes;
hybridizing the third oligonucleotide to the nucleic acid template strand at a location 3' to the sequence to which sequence Z hybridizes in the nucleic acid template strand; and
synthetically extending the 3' end of the third oligonucleotide by nucleic acid polymerization using the polymerase having strand displacement activity, thereby displacing from the nucleic acid template strand the first strand synthesized by extension of the first oligonucleotide primer.

6. The method of claim 1 or 2, wherein the nucleic acid template strand is one strand of a dsDNA, and wherein the method further comprises:
in the providing step, providing an osmolyte in the reaction mixture; and
chemically or physically denaturing the dsDNA before hybridizing the first oligonucleotide primer to the nucleic acid template strand.

7. The method of claim 1 or 2, wherein the nucleic acid template strand is ssDNA having a defined 3' end, and wherein the method further includes synthetically extending the 3' end of the nucleic acid template strand by nucleic acid polymerization to make an AT-rich sequence complementary to the sequence X of the first oligonucleotide primer.

8. The method of claim 1 or 2, wherein the nucleic acid template strand is one strand of a dsRNA, and wherein the method further includes the steps of:
in the providing step, providing an enzyme that has reverse transcriptase (RT) activity and a means for cleaving RNA,
before the hybridizing steps, chemically or physically denaturing the dsRNA to separate a first ssRNA strand that hybridizes to the first oligonucleotide primer and a second ssRNA strand that hybridizes to the second oligonucleotide primer,
hybridizing sequence Z of the first oligonucleotide primer to a complementary sequence in the first ssRNA strand that serves as the nucleic acid template strand,
hybridizing the second oligonucleotide primer to a complementary sequence in the second ssRNA strand,
using the RT activity to synthetically extend the 3' terminus of the first oligonucleotide primer hybridized to the first ssRNA strand and to extend the 3' terminus of the second oligonucleotide primer hybridized to the second ssRNA strand,
using the means for cleaving RNA to degrade the first ssRNA strand to make sequence Y accessible to hybridization with the second oligonucleotide primer, and
using the means for cleaving RNA to degrade the second ssRNA strand to make an extension product of the second oligonucleotide primer accessible to hybridization with the first oligonucleotide primer.

9. The method of any one of claims 1-8, wherein the providing step further includes an osmolyte in the reaction mixture.

10. The method of claim 9, wherein the osmolyte is betaine or trimethylamine N-oxide.

11. The method of any one of claims 1-10, wherein in the providing step the nucleic acid polymerase having strand displacement activity is a polymerase derived from a thermophilic organism.

12. The method of claim 11, wherein the nucleic acid polymerase h is a DNA polymerase derived from *Bacillus stearothermophilus* (*Bst*).

13. The method of any one of claims 1-12, wherein the AT-rich sequence X is made up of about 85% -100% A and T residues.

14. The method of any one of claims 1-13, wherein the polymerizing step h) is performed at about 65°C.

15. The method of any one of claims 1-14, wherein the providing step further provides a binding molecule that binds to the nucleic acid template strand and limits extension of the first oligonucleotide primer before the 5' end of the nucleic acid template strand.

16. The method of claim 15, wherein the binding molecule is an oligonucleotide that hybridizes to the nucleic acid template strand and includes at least one peptide nucleic acid (PNA), locked nucleic acid (LNA) or 2'-O-methyl ribonucleotide residue.

17. The method of claim 15, wherein the binding molecule comprises a nuclease activity.

18. The method of claim 3 or 8, wherein the nucleic acid polymerase having strand displacement activity also has reverse transcriptase (RT) activity and the means for cleaving RNA is an enzyme having RNase H activity.

19. The method of claim 4, wherein physically denaturing the nucleic acid template strand from the first strand includes raising the temperature of the mixture to a first temperature that separates the nucleic acid template strand and the first strand, and then cooling the mixture to a second temperature that does not denature a double-stranded nucleic acid made up of an extension product of the first oligonucleotide primer and an extension product of the second oligonucleotide primer.

20. The method of claim 4, wherein in step c) the 3' terminus of the nucleic acid template strand is not extended by the nucleic acid polymerase.

21. The method of claim 8, wherein physically denaturing the dsRNA to separate the first ssRNA strand and the second ssRNA strand includes raising the temperature of the mixture to a first temperature that denatures the dsRNA, and then cooling the mixture to a second temperature that does not denature a duplex made up of the first ssRNA strand and a strand made by synthetically extending the 3' terminus of the first oligonucleotide primer hybridized to the first ssRNA strand.

## Patentansprüche

1. Ein isothermales Nukleinsäureamplifikationsverfahren, das umfasst:
a) Bereitstellen einer Reaktionsmischung, die einschließt
i) einen Nukleinsäure-Matrizenstrang;
ii) Verlängerungsnukleotide;
iii) einen ersten Oligonukleotid-Primer, der eine 5'-terminale AT-reiche Sequenz X, die nicht komplementär ist zu einer Sequenz im Nukleinsäure-Matrizenstrang oder aus einer Sequenz im Nukleinsäure-Matrizenstrang besteht, und eine 3'-terminale Sequenz Z, die zu einer Sequenz in dem Nukleinsäure-Matrizenstrang komplementär ist, enthält, wobei die AT-reiche Sequenz X eine Länge von etwa 10-40 nt besitzt und zu 65-100 % aus A und T Resten besteht;
iv) ein zweiter Oligonukleotid-Primer, der aus einer Sequenz besteht, die in dem Nukleinsäure-Matrizenstrang enthalten ist; und
v) eine Nukleinsäure-Polymerase, die Strang-Verdrängungsaktivität besitzt;
b) Hybridisieren der Sequenz Z des ersten Oligonukleotid-Primers an eine komplementäre Sequenz in dem Nukleinsäure-Matrizenstrang, wobei, falls der Nukleinsäure-Matrizenstrang ein Strang einer doppelsträngigen Nukleinsäure ist, die doppelsträngige Nukleinsäure chemisch oder physikalisch denaturiert wird bevor der erste Oligonukleotid-Primer an den Nukleinsäure-Matrizenstrang hybridisiert wird;
c) synthetische Verlängerung des ersten Oligonukleotid-Primers durch Nukleinsäure-Polymerisation ausgehend von dem 3'-Ende der Sequenz Z, um Sequenz Y herzustellen, die zu mindestens einem Teil des Nukleinsäure-Matrizenstrangs komplementär ist, dadurch Ausbilden eines ersten Strangs einer doppelsträngigen Nukleinsäure, die isothermal amplifiziert wird;
d) mindestens teilweises Trennen der Sequenz Y von dem Nukleinsäure-Matrizenstrang;
e) Hybridisieren des zweiten Oligonukleotid-Primers an eine komplementäre Sequenz, die in Sequenz Y enthalten ist;
f) synthetische Verlängerung des 3'-Endes des zweiten Oligonukleotid-Primers durch Nukleinsäure-Polymerisation, dadurch Ausbilden eines zweiten Strangs der doppelsträngigen Nukleinsäure, die isothermal amplifiziert wird, wobei der zweite Strang eine AT-reiche Sequenz enthält, die zu der Sequenz X des ersten Nukleotid-Primers komplementär ist, dadurch Ausbilden einer AT-reichen Region der doppelsträngigen Nukleinsäure, die isothermal amplifiziert wird;
g) Hybridisieren des ersten Oligonukleotid-Primers an den zweiten Strang der doppelsträngigen Nukleinsäure, die isothermal unter Bedingungen amplifiziert wird, unter denen die AT-reiche Region der doppelsträngigen Nukleinsäure teilweise geöffnet ist, um den zweiten Strang für den ersten Oligonukleotid-Primer zugänglich zu machen; und
h) Polymerisieren eines Verlängerungsprodukts des ersten Oligonukleotid-Primers, der an den zweiten Strang hybridisiert ist, durch Verwendung der Nukleinsäure-Polymerase, die Strang-Verdrängungsaktivität besitzt, dadurch Verdrängen des ersten Strangs der doppelsträngigen Nukleinsäure und Durchführen von mindestens einem Amplifizierungszyklus unter isothermalen Bedingungen an der doppelsträngigen Nukleinsäure, die isothermal amplifiziert wird.

2. Das Verfahren nach Anspruch 1, wobei der Amplifizierungszyklus unter isothermalen Bedingungen ferner das Hybridisieren des zweiten Oligonukleotid-Primers an den ersten Strang, der durch das Polymerisieren entfernt wurde, um das Verlängerungsprodukt des ersten Oligonukleotid-Primers auszubilden, und das Verlängern des 3'-Endes des zweiten Oligonukleotid-Primers durch Nukleinsäure-Polymerisation, unter Verwendung des ersten Strangs als Matrize, einschließt.

3. Das Verfahren nach Anspruch 1 oder2, wobei der Nukleinsäure-Matrizenstrang ssRNA ist und wobei die Reaktionsmischung ferner ein Enzym mit Reverse Transkriptase (RT) Aktivität und ein Mittel zur RNA Spaltung enthält, wobei die RT Aktivität den ersten Oligonukleotid-Primer ausgehend vom 3'-Ende der Sequenz Z synthetisch verlängert, um Sequenz Y in dem ersten Strang herzustellen, und das Mittel zur RNA Spaltung den ssRNA Matrizenstrang während oder nach der Synthese des ersten Strangs abbaut.

4. Das Verfahren nach Anspruch 1 oder 2, wobei der Nukleinsäure-Matrizenstrang ssDNA ist und wobei das Verfahren ferner einen Schritt der chemischen oder physikalischen Denaturierung des Nukleinsäure-Matrizenstrangs von dem ersten Strang, der durch synthetische Verlängerung des ersten Oligonukleotid-Primers ausgehend vom 3'-Ende der Sequenz Z durch Nukleinsäure-Polymerisation hergestellt wurde, um Sequenz Y, die mindestens zu einem Teil zum Nukleinsäure-Matrizenstrang komplementär ist, herzustellen, einschließt.

5. Das Verfahren nach Anspruch 1 oder 2, wobei der Nukleinsäure-Matrizenstrang ssDNA
ist und wobei das Verfahren ferner umfasst:
Bereitstellen eines dritten Oligonukleotids, das Sequenz T einschließt, die an eine Sequenz in dem Nukleinsäure-Matrizenstrang hybridisiert, die 3' von der Sequenz lokalisiert ist, an welche Sequenz Z hybridisiert, in der Reaktionsmischung im Bereitstellungsschritt;
Hybridisieren des dritten Oligonukleotids an den Nukleinsäure-Matrizenstrang an einer Stelle 3' zu der Sequenz, an welche Sequenz Z an dem Nukleinsäure-Matrizenstrang hybridisiert; und
synthetische Verlängerung des 3'-Endes des dritten Oligonukleotids durch Nukleinsäure-Polymerisation unter Verwendung der Polymerase, die Strang-Verdrängungsaktivität besitzt, dadurch Verdrängen des ersten Strangs, der durch Verlängerung des ersten Oligonukleotid-Primers synthetisiert wurde, vom Nukleinsäure-Matrizenstrang.

6. Das Verfahren nach Anspruch 1 oder 2, wobei der Nukleinsäure-Matrizenstrang ein Strang einer dsDNA ist und wobei das Verfahren ferner umfasst:
Bereitstellen eines Osmolyts in der Reaktionsmischung im Bereitstellungsschritt; und
chemische oder physikalische Denaturierung der dsDNA vor der Hybridisierung des ersten Oligonukleotid-Primers an den Nukleinsäure-Matrizenstrang.

7. Das Verfahren nach Anspruch 1 oder 2, wobei der Nukleinsäure-Matrizenstrang ssDNA ist, die ein definiertes 3'-Ende besitzt, und wobei das Verfahren ferner das synthetische Verlängern des 3'-Endes des Nukleinsäure-Matrizenstrangs durch Nukleinsäure-Polymerisation einschließt, um eine AT-reiche Sequenz herzustellen, die zu der Sequenz X des ersten Oligonukleotid-Primers komplementär ist.

8. Das Verfahren nach Anspruch 1 oder 2, wobei der Nukleinsäure-Matrizenstrang ein Strang einer dsRNA ist und wobei das Verfahren ferner die Schritte einschließt:
Bereitstellen eines Enzyms, das Reverse Transkriptase (RT) Aktivität besitzt, und eines Mittels zur RNA Spaltung in dem Bereitstellungsschritt,
chemisches oder physikalisches Denaturieren der dsRNA, um einen ersten ssRNA Strang, der an den ersten Oligonukleotid-Primer hybridisiert, und einen zweiten ssRNA Strang, der an den zweiten Oligonukleotid-Primer hybridisiert, zu trennen vor den Hybridisierungsschritten,
Hybridisieren der Sequenz Z des ersten Oligonukleotid-Primers an eine komplementäre Sequenz in dem ersten ssRNA Strang, der als der Nukleinsäure-Matrizenstrang dient,
Hybridisieren des zweiten Oligonukleotid-Primers an eine komplementäre Sequenz in dem zweiten ssRNA Strang,
Verwenden der RT Aktivität, um das 3'-Ende des ersten Oligonukleotid-Primers, der an den ersten ssRNA Strang hybridisiert ist, zu verlängern und um das 3'-Ende des zweiten Oligonukleotid-Primers, der an den zweiten ssRNA Strang hybridisiert ist, synthetisch zu verlängern,
Verwenden des Mittels zur RNA Spaltung, um den ersten ssRNA Strang abzubauen, um Sequenz Y für die Hybridisierung mit dem zweiten Oligonukleotid-Primer zugänglich zu machen, und
Verwenden des Mittels zur RNA Spaltung, um den zweiten ssRNA Strang abzubauen, um ein Verlängerungsprodukt des zweiten Oligonukleotid-Primers für die Hybridisierung mit dem ersten Oligonukleotid-Primer zugänglich zu machen.

9. Das Verfahren nach einem der Ansprüche 1-8, wobei der Bereitstellungsschritt ferner einen Osmolyt in der Reaktionsmischung einschließt.

10. Das Verfahren nach Anspruch 9, wobei der Osmolyt Betain oder Trimethylamin-N-oxid ist.

11. Das Verfahren nach einem der Ansprüche 1-10, wobei in dem Bereitstellungsschritt die Nukleinsäure-Polymerase, die Strang-Verdrängungsaktivität besitzt, eine Polymerase ist, die von einem thermophilen Organismus stammt.

12. Das Verfahren nach Anspruch 11, wobei die Nukleinsäure-Polymerase eine DNA Polymerase ist, die von *Bacillus stearothermophilus* (Bst) stammt.

13. Das Verfahren nach einem der Ansprüche 1-12, wobei die AT-reiche Sequenz X zu etwa 85 %-100 % aus A und T Resten besteht.

14. Das Verfahren nach einem der Ansprüche 1-13, wobei der Polymerisierungsschritt h) bei etwa 65 °C durchgeführt wird.

15. Das Verfahren nach einem der Ansprüche 1-14, wobei der Bereitstellungsschritt ferner einen Bindungsmolekül bereitstellt, das an den Nukleinsäure-Matrizenstrang bindet und die Verlängerung des ersten Oligonukleotid-Primers vor dem 5'-Ende des Nukleinsäure-Matrizenstrangs einschränkt.

16. Das Verfahren nach Anspruch 15, wobei das Bindungsmolekül ein Oligonukleotid ist, das an den Nukleinsäure-Matrizenstrang bindet und mindestens eine Peptidnukleinsäure (PNA), verbrückte Nukleinsäure (LNA) oder einen 2'-O-Methylribonukleotid-Rest enthält.

17. Das Verfahren nach Anspruch 15, wobei das Bindungsmolekül eine Nuklease-Aktivität umfasst.

18. Das Verfahren nach Anspruch 3 oder 8, wobei die Nukleinsäure-Polymerase, die Strang-Verdrängungsaktivität besitzt, ebenfalls Reverse Transkriptase (RT) Aktivität besitzt und das Mittel zur RNA Spaltung ein Enzym ist, das RNase H Aktivität besitzt.

19. Das Verfahren nach Anspruch 4, wobei die physikalische Denaturierung des Nukleinsäure-Matrizenstrangs von dem ersten Strang die Erhöhung der Temperatur der Mischung auf eine ersten Temperatur, die den Nukleinsäure-Matrizenstrang und den ersten Strang trennt, und dann das Abkühlen der Mischung auf eine zweite Temperatur, die eine doppelsträngige Nukleinsäure, die aus einem Verlängerungsprodukt des ersten Oligonukleotid-Primers und einem Verlängerungsprodukt des zweiten Oligonukleotid-Primers besteht, nicht denaturiert, einschließt.

20. Das Verfahren nach Anspruch 4, wobei in Schritt c) das 3'-Ende des Nukleinsäure-Matrizenstrangs nicht durch die Nukleinsäure-Polymerase verlängert wird.

21. Das Verfahren nach Anspruch 8, wobei das physikalische Denaturieren der dsRNA, um den ersten ssRNA Strang und den zweiten ssRNA Strang zu trennen, die Erhöhung der Temperatur der Mischung auf eine erste Temperatur, die den dsRNA Strang denaturiert, und dann das Abkühlen der Mischung auf eine zweite Temperatur, die eine Duplex, die aus dem ersten ssRNA Strang und einem Strang, der durch synthetische Verlängerung des 3'-Endes des ersten Oligonukleotid-Primers, der an den ersten ssRNA Strang hybridisiert ist, aufgebaut ist, nicht denaturiert, einschließt.

## Revendications

1. Procédé d'amplification d'acide nucléique isotherme comprenant :
a) la fourniture d'un mélange réactionnel qui comprend
i) un brin matrice d'acide nucléique ;
ii) des nucléotides d'extension ;
iii) une première amorce oligonucléotidique qui contient une séquence X riche en AT d'extrémité 5' qui n'est pas complémentaire à ou qui consiste en une séquence dans le brin matrice d'acide nucléique et une séquence Z d'extrémité 3' qui est complémentaire à une séquence dans le brin matrice d'acide nucléique, où la séquence X riche en AT a une longueur d'environ 10 à 40 nt et est faite de 65 % à 100 % de résidus A et T;
iv) une deuxième amorce oligonucléotidique consistant en une séquence contenue dans le brin matrice d'acide nucléique ; et
v) une polymérase d'acide nucléique ayant une activité de déplacement de brin ;
b) l'hybridation de la séquence Z de la première amorce oligonucléotidique à une séquence complémentaire dans le brin matrice d'acide nucléique, où, si le brin matrice d'acide nucléique est un brin d'un acide nucléique bicaténaire, alors l'acide nucléique bicaténaire est dénaturé chimiquement ou physiquement avant l'hybridation de la première amorce oligonucléotidique au brin matrice d'acide nucléique ;
c) l'extension de manière synthétique de la première amorce nucléotidique à partir de l'extrémité 3' de la séquence Z par polymérisation de l'acide nucléique pour préparer une séquence Y qui est complémentaire à au moins une partie du brin matrice d'acide nucléique, en formant ainsi un premier brin d'un acide nucléique bicaténaire qui est amplifié de manière isotherme ;
d) la séparation au moins partielle de la séquence Y du brin matrice d'acide nucléique ;
e) l'hybridation de la deuxième amorce oligonucléotidique à une séquence complémentaire contenue dans la séquence Y ;
f) l'extension de manière synthétique de l'extrémité 3' de la deuxième amorce oligonucléotidique par polymérisation de l'acide nucléique, en formant ainsi un deuxième brin de l'acide nucléique bicaténaire qui est amplifié de manière isotherme, dans laquelle le deuxième brin contient une séquence riche en AT complémentaire à une séquence X de la première amorce oligonucléotidique, en formant ainsi une région riche en AT de l'acide nucléique bicaténaire qui est amplifié de manière isotherme ;
g) l'hybridation de la première amorce oligonucléotidique au deuxième brin de l'acide nucléique bicaténaire qui est amplifié de manière isotherme dans des conditions dans lesquelles la région riche en AT de l'acide nucléique bicaténaire est partiellement ouverte pour rendre accessible le deuxième brin à la première amorce oligonucléotidique ; et
h) la polymérisation d'un produit d'extension de la première amorce oligonucléotidique hybridée au deuxième brin en utilisant la polymérase d'acide nucléique ayant une activité de déplacement de brin, en déplaçant ainsi le premier brin de l'acide nucléique bicaténaire et en réalisant au moins un cycle d'amplification dans des conditions isothermes sur l'acide nucléique bicaténaire qui est amplifié de manière isotherme.

2. Procédé selon la revendication 1, dans lequel le cycle d'amplification dans des conditions isothermes inclut en outre l'hybridation de la deuxième amorce oligonucléotidique au premier brin qui a été déplacé par polymérisation pour former le produit d'extension de la première amorce oligonucléotidique, et l'extension de l'extrémité 3' de la deuxième amorce oligonucléotidique par polymérisation de l'acide nucléique en utilisant le premier brin en tant qu'une matrice.

3. Procédé selon la revendication 1 ou 2, dans lequel le brin matrice d'acide nucléique est un ARN monocaténaire, et où le mélange réactionnel inclut en outre une enzyme avec une activité de transcriptase inverse (RT) et des moyens pour cliver l'ARN, grâce à quoi l'activité de RT allonge de manière synthétique la première amorce oligonucléotidique à partir de l'extrémité 3' de la séquence Z pour préparer une séquence Y dans le premier brin et les moyens pour cliver l'ARN dégradent le brin matrice d'ARN monocaténaire durant ou après la synthèse du premier brin.

4. Procédé selon la revendication 1 ou 2, dans lequel le brin matrice d'acide nucléique est un ADN monocaténaire, et où le procédé inclut en outre une étape de dénaturation chimique ou physique du brin matrice d'acide nucléique à partir du premier brin préparé par l'extension de manière synthétique de la première amorce oligonucléotidique à partir de l'extrémité 3' de la séquence Z par polymérisation d'acide nucléique pour préparer une séquence Y qui est complémentaire à au moins une partie du brin matrice d'acide nucléique.

5. Procédé selon la revendication 1 ou 2, dans lequel le brin matrice d'acide nucléique est un ADN monocaténaire, et où le procédé comprend en outre :
dans l'étape de fourniture, la fourniture dans le mélange réactionnel d'un troisième oligonucléotide qui inclut une séquence T qui s'hybride à une séquence dans le brin matrice d'acide nucléique situé en 3' de la séquence à laquelle la séquence Z s'hybride ;
l'hybridation du troisième oligonucléotide au brin matrice d'acide nucléique à un emplacement en 3' de la séquence à laquelle la séquence Z s'hybride dans le brin matrice d'acide nucléique ; et
l'extension de manière synthétique de l'extrémité 3' du troisième oligonucléotide par polymérisation de l'acide nucléique en utilisant la polymérase ayant une activité de déplacement de brin, en déplaçant ainsi du brin matrice d'acide nucléique le premier brin synthétisé par l'extension de la première amorce oligonucléotidique.

6. Procédé selon la revendication 1 ou 2, dans lequel le brin matrice d'acide nucléique est un brin d'un ADN bicaténaire, et où le procédé comprend en outre :
dans l'étape de fourniture, la fourniture d'un osmolyte dans le mélange réactionnel ; et
la dénaturation chimique ou physique de l'ADN bicaténaire avant l'hybridation de la première amorce oligonucléotidique au brin matrice d'acide nucléique.

7. Procédé selon la revendication 1 ou 2, dans lequel le brin matrice d'acide nucléique est un ADN monocaténaire ayant une extrémité 3' définie, et où le procédé inclut en outre l'extension de manière synthétique de l'extrémité 3' du brin matrice d'acide nucléique par polymérisation de l'acide nucléique pour préparer une séquence riche en AT complémentaire à la séquence X de la première amorce oligonucléotidique.

8. Procédé selon la revendication 1 ou 2, dans lequel le brin matrice d'acide nucléique est un brin d'un ARN bicaténaire, et où le procédé inclut en outre les étapes de :
dans l'étape de fourniture, la fourniture d'une enzyme qui possède une activité de transcriptase inverse (RT) et des moyens pour cliver l'ARN,
avant les étapes d'hybridation, la dénaturation chimique ou physique de l'ARN bicaténaire pour séparer un premier brin d'ARN monocaténaire qui s'hybride à la première amorce nucléotidique et un deuxième brin d'ARN monocaténaire qui s'hybride à la deuxième amorce oligonucléotidique,
l'hybridation de la séquence Z de la première amorce oligonucléotidique à une séquence complémentaire dans le premier brin d'ARN monocaténaire qui sert de brin matrice d'acide nucléique,
l'hybridation de la deuxième amorce oligonucléotidique à une séquence complémentaire dans le deuxième brin d'ARN monocaténaire,
l'utilisation de l'activité RT pour allonger de manière synthétique l'extrémité 3' de la première amorce oligonucléotidique hybridée au premier brin d'ARN monocaténaire et pour allonger l'extrémité 3' de la deuxième amorce oligonucléotidique hybridée au deuxième brin d'ARN monocaténaire,
l'utilisation des moyens pour cliver l'ARN pour dégrader le premier brin d'ARN monocaténaire pour rendre une séquence Y accessible à l'hybridation avec la deuxième amorce oligonucléotidique, et
l'utilisation des moyens pour cliver l'ARN pour dégrader le deuxième brin d'ARN monocaténaire pour rendre un produit d'extension de la deuxième amorce oligonucléotidique accessible à l'hybridation avec la première amorce oligonucléotidique.

9. Procédé selon l'une quelconque des revendications 1 à 8, dans lequel l'étape de fourniture inclut en outre un osmolyte dans le mélange réactionnel.

10. Procédé selon la revendication 9, dans lequel l'osmolyte est la bétaïne ou la N-oxyde de triméthylamine.

11. Procédé selon l'une quelconque des revendications 1 à 10, dans lequel dans l'étape de fourniture, la polymérase d'acide nucléique ayant une activité de déplacement de brin est une polymérase dérivé d'un organisme thermophile.

12. Procédé selon la revendication 11, dans lequel la polymérase d'acide nucléique est une ADN polymérase dérivée de *Bacillus stearothermophilus* (*Bst*).

13. Procédé selon l'une quelconque des revendications 1 à 12, dans lequel la séquence X riche en AT est faite d'environ 85 % à 100 % de résidus A et T.

14. Procédé selon l'une quelconque des revendications 1 à 13, dans lequel l'étape de polymérisation h) est réalisée à environ 65°C.

15. Procédé selon l'une quelconque des revendications 1 à 14, dans lequel l'étape de fourniture fournit en outre une molécule de liaison qui se lie au brin matrice d'acide nucléique et limite l'extension de la première amorce oligonucléotidique avant l'extrémité 5' du brin matrice d'acide nucléique.

16. Procédé selon la revendication 15, dans lequel la molécule de liaison est un oligonucléotide qui s'hybride au brin matrice d'acide nucléique et inclut au moins un acide nucléique peptidique (PNA), un acide nucléique bloqué (LNA) ou un résidu 2'-O-méthyl ribonucléotidique.

17. Procédé selon la revendication 15, dans lequel la molécule de liaison comprend une activité de nucléase.

18. Procédé selon la revendication 3 ou 8, dans lequel la polymérase d'acide nucléique ayant une activité de déplacement de brin possède également une activité de transcriptase inverse (RT) et les moyens pour cliver l'ARN sont une enzyme ayant une activité de RNase H.

19. Procédé selon la revendication 4, dans lequel la dénaturation physique du brin matrice d'acide nucléique à partir du premier brin inclut l'élévation de la température du mélange à une première température qui sépare le brin matrice d'acide nucléique et le premier brin, et ensuite le refroidissement du mélange à une deuxième température qui ne dénature pas un acide nucléique bicaténaire fait d'un produit d'extension de la première amorce oligonucléotidique et d'un produit d'extension de la deuxième amorce oligonucléotidique.

20. Procédé selon la revendication 4, dans lequel dans l'étape c) l'extrémité 3' du brin matrice d'acide nucléique n'est pas allongée par la polymérase d'acide nucléique.

21. Procédé selon la revendication 8, dans lequel la dénaturation physique de l'ARN bicaténaire pour séparer le premier brin d'ARN monocaténaire et le deuxième brin d'ARN monocaténaire inclut l'élévation de la température du mélange à une première température qui dénature l'ARN bicaténaire, et ensuite le refroidissement du mélange à une deuxième température qui ne dénature pas un duplex fait du premier brin d'ARN monocaténaire et d'un brin préparé par l'extension de manière synthétique de l'extrémité 3' de la première amorce oligonucléotidique hybridée au premier brin d'ARN monocaténaire.


### SEQUENCE LISTING

<110> Gen-Probe Incorporated
   Becker, Michael M.
   Livezey, Kristin W.
<120> Methods, compositions and Kits for Isothermal Amplification of Nucleic Acids
<130> GP174-PCT
<140> To Be Assigned
   <141> 2006-09-06
<150> US 60/714,281
   <151> 2005-09-06
<150> US 60/722,028
   <151> 2005-09-29
<160> 17
<170> PatentIn version 3.3
<210> 1
   <211> 120
   <212> DNA
   <213> Artificial
<220>
   <223> synthetic oligonucleotide
<400> 1
   atggttacga attagtcact ccgtggatag agtcgctaga cagataaagc aagtaggtat 60
   caacggactg aggggcagca cactacaagc ttagaagata gagagggatt aaaaaaaaaa 120
<210> 2
   <211> 20
   <212> DNA
   <213> Artificial
<220>
   <223> synthetic oligonucleotide primer
<400> 2
   ctaagcttgt agtgtgctgc 20
<210> 3
   <211> 26
   <212> DNA
   <213> Artificial
<220>
   <223> synthetic oligonucleotide probe
<400> 3
   gcaagtaggt tatcaacgga ctgagg 26
<210> 4
   <211> 120
   <212> DNA
   <213> Artificial
<220>
   <223> Synthetic oligonucleotide
<400> 4
   tttttttttt aatccctctc tatcttctaa gcttgtagtg tgctgcccct cagtccgttg 60
   atacctactt gctttatctg tctagcgact ctatccacgg agtgactaat tcgtaaccat 120
<210> 5
   <211> 20
   <212> DNA
   <213> Artificial
<220>
   <223> synthetic oligonucleotide primer
<400> 5
   tttttttttt aatccctctc 20
<210> 6
   <211> 18
   <212> DNA
   <213> Artificial
<220>
   <223> synthetic oligonucleotide
<400> 6
   atagagtcgc tagacaga 18
<210> 7
   <211> 120
   <212> DNA
   <213> Artificial
<220>
   <223> synthetic oligonucleotide
<400> 7
   aaaaaaattt ttttcgagcc gtatagagtc gctagacaga taaagcaagt aggtatcaac 60
   ggactgaggg gcagcacact acaagcttag aagatagaga gggattaaaa aaaaaaaaaa 120
<210> 8
   <211> 120
   <212> DNA
   <213> Artificial
<220>
   <223> synthetic oligonucleotide
<400> 8
   tttttttttt ttttaatccc tctctatctt ctaagcttgt agtgtgctgc ccctcagtcc 60
   gttgatacct acttgcttta tctgtctagc gactctatac ggctcgaaaa aaattttttt 120
<210> 9
   <211> 35
   <212> DNA
   <213> Artificial
<220>
   <223> Synthetic oligonucleotide
<400> 9
   tttttttttt aatccctctc tatcttctaa gcttg 35
<210> 10
   <211> 83
   <212> DNA
   <213> Artificial
<220>
   <223> Synthetic oligonucleotide
<400> 10
   tttttttttt aatccctctc tatcttctaa gcttgtagtg tgctctagcg actctatcca 60
   cggagtgact aattcgtaac cat 83
<210> 11
   <211> 83
   <212> DNA
   <213> Artificial
<220>
   <223> Synthetic oligonucleotide
<400> 11
   atggttacga attagtcact ccgtggatag agtcgctaga gcacactaca agcttagaag 60
   atagagaggg attaaaaaaa aaa 83
<210> 12
   <211> 120
   <212> DNA
   <213> Artificial
<220>
   <223> Synthetic oligonucleotide
<400> 12
   atggttacga ctgatcatca tagagtcgct agacagataa agcaagtagg tatcaacgga 60
   ctgaggggat agtctacatg gcagcacact acaagcttag acatgaacgt actgtcaagt 120
<210> 13
   <211> 120
   <212> DNA
   <213> Artificial
<220>
   <223> synthetic oligonucleotide
<400> 13
   acttgacagt acgttcatgt ctaagcttgt agtgtgctgc catgtagact atcccctcag 60
   tccgttgata cctacttgct ttatctgtct agcgactcta tgatgatcag tcgtaaccat 120
<210> 14
   <211> 120
   <212> DNA
   <213> Artificial
<220>
   <223> Synthetic oligonucleotide
<400> 14
   aaaaaaattt ttttcgagcc gtatagagtc gctagacaga taaagcaagt aggtatcaac 60
   ggactgaggg gcagcacact acaagcttag aagatagaga gggattaaaa aaaaaaaaaa 120
<210> 15
   <211> 120
   <212> DNA
   <213> Artificial
<220>
   <223> Synthetic oligonucleotide
<400> 15
   tttttttttt ttttaatccc tctctatctt ctaagcttgt agtgtgctgc ccctcagtcc 60
   gttgatacct acttgcttta tctgtctagc gactctatac ggctcgaaaa aaattttttt 120
<210> 16
   <211> 81
   <212> DNA
   <213> Artificial
<220>
   <223> synthetic oligonucleotide
<400> 16
   atagagtcgc tagacagata aagcaagtag gtatcaacgg actgagggga tagtctacat 60
   ggcagcacac tacaagctta g 81
<210> 17
   <211> 81
   <212> DNA
   <213> Artificial
<220>
   <223> Synthetic oligonucleotide
<400> 17
   ctaagcttgt agtgtgctgc catgtagact atcccctcag tccgttgata cctacttgct 60
   ttatctgtct agcgactcta t 81